# EUROPEAN PATENT APPLICATION

(11) **EP 4 675 642 A1**
(43) Date of publication of application: **07.01.2026**
(21) Application number: 24763982.6
(22) Date of filing: 28.02.2024
(51) Int. Cl.: G16H 50/70, G16H 30/40

(54) **INFORMATION PROCESSING SYSTEM, METHOD FOR CONTROLLING INFORMATION PROCESSING SYSTEM, CONTROL PROGRAM, AND RECORDING MEDIUM**

(30) Priority: 28.02.2023 JP 2023030323
(71) Applicant: KYOCERA Corporation, Kyoto 612-8501 (JP)
(72) Inventor: WATANABE, Kenichi, Kyoto-shi, Kyoto 612-8501 (JP); KYOMOTO, Masayuki, Kyoto-shi, Kyoto 612-8501 (JP); KAMEI, Kentaro, Kyoto-shi, Kyoto 612-8501 (JP)
(74) Representative: TBK
(86) International application number: PCT/JP2024/007343
(87) International publication number: WO 2024/181503

(57) **Abstract**

To support planning of a surgical treatment suitable for an affected bone of a subject. An information processing system includes a first estimator and an outputter. The first estimator inputs input information including a first image showing an affected area including a target bone of a subject in which an implant is placed into a trained first learning model, and estimates first estimation information regarding at least any of a bone density, a bone mass, and a bone quality of the target bone. The outputter outputs the first estimation information.

## Description

### TECHNICAL FIELD

The present disclosure relates to an information processing system that estimates the prognosis of a subject to whom an implant placement surgery has been applied, a control method thereof, and the like.

### BACKGROUND OF INVENTION

Patent Document 1 discloses a technique for diagnosing a patient's risk of implant-related revision.

### CITATION LIST

### PATENT LITERATURE

Patent Document 1: JP 2020-507783 A

### SUMMARY

In one aspect of the present disclosure, an information processing system includes an acquirer configured to acquire input information including a first image showing at least a part of a target bone of a subject in which a first implant is placed, a first estimator configured to input the input information into a first learning model trained using first training data including a second image showing at least a part of a bone of an animal including a person, and bone information regarding at least any of a bone density, a bone mass, and a bone quality of the bone, and estimate first estimation information regarding at least any of a bone density, a bone mass, and a bone quality of the target bone, and an outputter configured to output the first estimation information.

In one aspect of the present disclosure, a method for controlling an information processing system includes acquiring input information including a first image showing at least a part of a target bone of a subject in which a first implant is placed, and inputting the input information into a learning model trained using first training data including a second image showing at least a part of a bone of an animal including a person, and bone information regarding at least any of a bone density, a bone mass, and a bone quality of the bone, estimating first estimation information regarding at least any of a bone density, a bone mass, and a bone quality of the target bone, and outputting the first estimation information.

The information processing system according to each aspect of the present disclosure may be implemented by a computer. In such a case, a control program of an information processing system that implements the information processing system on a computer by causing the computer to operate as each component (a software element) of the information processing system, and a computer-readable recording medium on which the control program is recorded, are also included in the scope of the present disclosure.

### BRIEF DESCRIPTION OF THE DRAWINGS

FIG. 1 is a diagram illustrating a configuration example of an information processing system according to one aspect of the present disclosure.
FIG. 2 is a diagram illustrating a configuration example of an information processing system according to another aspect of the present disclosure.
FIG. 3 is a block diagram illustrating an example of a configuration of an information processing device according to one aspect of the present disclosure.
FIG. 4 is a view illustrating an example of a plurality of sites in a target bone.
FIG. 5 is a view illustrating an example of a plurality of sites in a target bone.
FIG. 6 is a diagram illustrating an example of a configuration of a first learning model executed by an identifier.
FIG. 7 is a flowchart illustrating an example of a flow of training processing by a trainer.
FIG. 8 is a flowchart illustrating an example of a flow of processing performed by the information processing device.
FIG. 9 is a block diagram illustrating an example of a configuration of an information processing device according to one aspect of the present disclosure.
FIG. 10 is a view illustrating an example of a plurality of sites in a target bone.
FIG. 11 is a view illustrating an example of a plurality of sites in a target bone.
FIG. 12 is a view illustrating an example of a plurality of sites in a target bone.
FIG. 13 is a flowchart illustrating another example of a flow of training processing by a trainer.
FIG. 14 is a diagram illustrating a configuration example of an information processing system according to one aspect of the present disclosure.
FIG. 15 is a block diagram illustrating an example of a configuration of an information processing device according to one aspect of the present disclosure.
FIG. 16 is a diagram illustrating an example of a configuration of a learning model executed by an estimator according to one aspect of the present disclosure.
FIG. 17 is a flowchart illustrating an example of a flow of training processing by a trainer according to one aspect of the present disclosure.
FIG. 18 is a flowchart illustrating an example of a flow of processing performed by an information processing device according to one aspect of the present disclosure.
FIG. 19 is a diagram illustrating a configuration example of an information processing system according to another aspect of the present disclosure.

### DESCRIPTION OF EMBODIMENTS

Embodiments according to the present disclosure will be described below. Hereinafter, a case in which a subject to which a surgical treatment method is to be applied is a human (that is, a "subject person") will be described in an example, but the subject is not limited to a human. That is, the "subject" according to the present disclosure may be, for example, a mammal other than a human, such as an equine, feline, canine, bovine, or porcine mammal. When any embodiment out of the following embodiments is applicable to any of such animals, the present disclosure also includes the embodiment in which the "subject," "patient," and "person" are replaced with "animal".

### First Embodiment

An information processing device 1 according to one embodiment of the present disclosure is described in an example below in detail.

The information processing device 1 outputs first estimation information estimated using a first learning model based on input information including a first image showing an affected area of a subject. In this case, the affected area includes at least a part of a bone in which an implant (first implant) is placed (hereinafter, referred to as a "target bone"). The first estimation information is information regarding training data of the first learning model, and includes, for example, bone information regarding the subject's bone state. The bone information may include, for example, at least one selected from the group consisting of a presence or absence of a fracture, a likelihood of osteoporosis, a drug efficacy, an incident occurrence, a bone density, a bone mass, a bone quality, a trabecular number, a trabecular spacing, a trabecular width, a trabecular orientation, a bone connectivity density, and a trabecular bone score. Alternatively, bone strength may be analyzed based on the state of cortical bone and/or cancellous bone and the resulting data may be used as bone information. The first estimation information may include bone information regarding at least any of a bone density, a bone mass, and a bone quality for a partial region of the target bone in which the implant is placed. The affected area may refer to a region with disease or injury. In this case, the affected area includes a region with previous disease or injury.

The likelihood of osteoporosis includes, for example, "no osteoporosis," "suspected osteoporosis," or "with osteoporosis," based on at least one selected from the group consisting of the presence or absence of a fracture, the likelihood of a fracture, and a change in bone density. More specifically, as the likelihood of osteoporosis, when there is no disease resulting in bone mass reduction and no secondary osteoporosis is observed, and there is a fracture or a high likelihood of fracture, primary osteoporosis may be indicated. The drug efficacy may include, for example, the name of a medication that improves the bone state when taken and/or administered for a certain period of time, or may include bone information including at least one selected from the group consisting of the bone density, the bone mass, and the state of the trabecular bone after a certain period of time has elapsed. The incident occurrence includes, for example, loosening of an implant, dislodgement of an implant, or infection around an implant. Examples of the implant include an artificial hip joint, an artificial knee joint, a spinal implant, a bolt inserted into a bone, or a dental implant.

The first image showing at least a part of the bone in which the implant is placed can be, for example, an image showing various implants to be described below and a part of the bone around the implants. As the first image, for example, at least one selected from the group consisting of an X-ray image, a computed tomography (CT) image, a magnetic resonance imaging (MRI) image, a positron emission tomography (PET) image, a dual-energy X-ray absorptiometry (DXA) image, and an ultrasound image can be used, but the present disclosure is not limited thereto. The first image may be, for example, an inspection apparatus image acquired from an inspection apparatus (for example, an X-ray inspection apparatus), or an image in which noise has been reduced from a captured inspection apparatus image. For noise reduction, for example, machine learning can be used. The information processing device 1 may acquire a first image showing a bone of the subject, from an image management device 5. The X-ray images may include panoramic X-ray images, for example, used in dentistry. A panoramic X-ray image may be, for example, an image that includes a plurality of teeth (for example, all teeth). The first image may include, for example, at least any of an inspection apparatus image acquired from an inspection apparatus (for example, an X-ray inspection apparatus), an image in which noise has been reduced from the inspection apparatus image, and an image obtained by digitizing an X-ray film output from the inspection apparatus. The first image may be, for example, an inspection apparatus image stored in an external storage terminal and an image in which noise has been reduced from the inspection apparatus image. As the first image showing the implant and bone, for example, an image obtained by applying image processing to an inspection apparatus image to improve the appearance of the bone around the implant may be used. The first image may be, for example, an image captured by irradiating a part of the skeleton with plain X-rays. The first image may be, for example, an image showing the entire bone, or an image showing at least a part of the bone. The first image may be an image showing a trabecular bone. The captured site of the first image may include, for example, at least a part of a head, a neck, a chest, a waist, a hip joint, a knee joint, an ankle joint, a foot, a toe, a shoulder joint, an elbow joint, a wrist joint, a hand, fingers, and a jaw joint. Note that the type of the captured site of the first image is not limited thereto. The X-ray image may be a front image showing the target site irradiated with plain X-rays from the front (for example, an image obtained by irradiating the target site with X-rays in the front-rear direction), or a side image showing the target site from the side (for example, an image obtained by irradiating the target site with X-rays in the left-right direction). The X-ray image may be an image showing the cortical bone and/or the cancellous bone. As the X-ray image, for example, a frontal chest X-ray image including a person's chest or a frontal lumbar X-ray image including a person's waist can be used. The chest X-ray image is, for example, an image showing at least one selected from the group consisting of ribs, a clavicle, and a sternum. The lumbar X-ray image is, for example, an image showing at least one selected from the group consisting of lumbar vertebrae, a pelvis, and a femur. The first image is not limited to the waist or the chest, and, for example, an image showing teeth, a jaw, an arm, a hand, a shoulder joint, a knee joint, a heel, a skull, or foot bones can be used. Note that when a CT image is used as the first image, information regarding the trabecular bone based on a three-dimensionally constructed image may be used, or information regarding the trabecular bone based on an image captured two-dimensionally can be used.

A measured value obtained by actually measuring the bone density from at least one selected from the group consisting of, for example, a hand, lumbar vertebrae, a proximal femur, a tibia, a heel, and an arm (for example, a radius) can be used for the bone density of the bone used in the first learning model. For example, a single energy X-ray absorptiometry method, a dual-energy X-ray absorptiometry (DXA) method, an ultrasound method, or a quantitative computed tomography (CT) method can be used for the measurement of the bone density. In a DXA apparatus that measures a bone density using the DXA method, when the bone density of the lumbar vertebrae is measured, X-rays are emitted to the lumbar vertebrae of a test subject from the front of the lumbar vertebrae. In the DXA apparatus, when the bone density of the proximal femur is measured, X-rays are emitted to the proximal femur of the test subject from the front of the proximal femur. Here, the "front of the lumbar vertebrae" and the "front of the proximal femur" are intended to be directions correctly facing sites to be captured such as the lumbar vertebrae and the proximal femur, and may be the ventral side of the test subject's body or the back side of the test subject. Note that the proximal femur includes, for example, at least one site selected from the group consisting of the neck, the trochanter, the diaphysis, and the entire proximal femur (neck, trochanter, and diaphysis). In the MD method, for example, the hand is irradiated with X-rays.

As the bone density of the bone used in the first learning model, the patient's bone density estimated may be used. The patient's bone density may be estimated, for example, by inputting a second image showing the patient's bone into a trained estimation model that has been machine-trained using training data including at least one image selected from the group consisting of an X-ray image, a CT image, an MRI image, and an ultrasound image showing the bone, and the actually measured bone density of the bone. As the bone density of the bone used in the first learning model, the patient's future and/or past bone density predicted may be used. The patient's future and/or past bone density may be predicted, for example, by inputting a second image showing the patient's bone into a prediction model that predicts future and/or past bone density from an image, which has been machine-trained using training data (learning data) including at least one image selected from the group consisting of an X-ray image, a CT image, an MRI image, and an ultrasound image showing the bone, and the actually measured bone density of the bone. The prediction model can use a trained model that has been machine-trained using training data that includes images showing the patient's bone and the bone density actually measured at a point in time that is a predetermined period (for example, three months, six months, one year, three years, five years, or the like) different from a point in time at which the images are captured. The prediction result may be a result after a period shorter than the above-mentioned predetermined period, a result after a period longer than the above-mentioned predetermined period, or a result after a period equal to the above-mentioned predetermined period. The bone density of the bone may be estimated for a specific portion of a predetermined region of the bone (for example, a part of the vertebral body and a part of the femur) or may be estimated separately for a plurality of specific portions.

The bone may be, for example, a single bone, or a skeleton constituted by a plurality of bones.

As the second image, for example, at least one selected from the group consisting of an X-ray image, a CT image, an MRI image, a PET image, a DXA image, and an ultrasound image can be used, but the present disclosure is not limited thereto. The second image may be, for example, an inspection apparatus image acquired from an inspection apparatus (for example, an X-ray inspection apparatus), or an image in which noise has been reduced from a captured inspection apparatus image. For noise reduction, for example, machine learning can be used. The X-ray images may include panoramic X-ray images, for example, used in dentistry. A panoramic X-ray image may be, for example, an image that includes a plurality of teeth (for example, all teeth). The second image may include, for example, at least any of an inspection apparatus image acquired from an inspection apparatus (for example, an X-ray inspection apparatus), an image in which noise has been reduced from the inspection apparatus image, and an image obtained by digitizing an X-ray film output from the inspection apparatus. The second image may be, for example, an inspection apparatus image stored in an external storage terminal and an image in which noise has been reduced from the inspection apparatus image. As the second image showing the implant (second implant) and bone, a processed image may be used. The processed image may be obtained, for example, by applying image processing to an inspection apparatus image to improve the appearance of the bone around the implant. The second image may be, for example, an image captured by irradiating a part of the skeleton with plain X-rays. The second image may be, for example, an image showing the entire bone, or an image showing at least a part of the bone. The captured site of the second image may include, for example, at least a part of a head, a neck, a chest, a waist, a hip joint, a knee joint, an ankle joint, a foot, a toe, a shoulder joint, an elbow joint, a wrist joint, a hand, fingers, and a jaw joint. Note that the type of the captured site of the second image is not limited thereto. The X-ray image may be a front image showing the target site irradiated with plain X-rays from the front (for example, an image obtained by irradiating the target site with X-rays in the front-rear direction), or a side image showing the target site from the side (for example, an image obtained by irradiating the target site with X-rays in the left-right direction). The X-ray image may be an image showing at least any of the trabecular bone, the cortical bone, and the cancellous bone. As the X-ray image, for example, a frontal chest X-ray image including a person's chest or a frontal lumbar X-ray image including a person's waist can be used. The chest X-ray image is, for example, an image showing at least one selected from the group consisting of ribs, a clavicle, and a sternum. The lumbar X-ray image is, for example, an image showing at least one selected from the group consisting of lumbar vertebrae, a pelvis, and a femur. The second image is not limited to the waist or the chest, and, for example, an image showing teeth, a jaw, an arm, a hand, a shoulder joint, a knee joint, a heel, a skull, or foot bones can be used. Note that when a CT image is used as the second image, information regarding the trabecular bone based on a three-dimensionally constructed image may be used, or information regarding the trabecular bone based on an image captured two-dimensionally can be used.

The bone mass is an index related to bone density and is a concept that includes bone density. The bone mass may be the sum of bone mineral and bone matrix protein. In the present disclosure, the bone mass may be an index related to bone density, and the bone mass is an amount of bone tissue in the skeleton. The bone quality can be based on, for example, at least one selected from the group consisting of a statistical property of the bone, a geometric property of the bone, a mechanical property of the bone, and a chemical property of the bone. The bone mass information may be information measured by a bone density measurement device such as DXA, or may be information obtained by estimating a bone density from an X-ray image using a trained parameter. The bone quality may include information regarding the subject's attributes to be described below. The bone quality usable can be based on at least one selected from the group consisting of, for example, a bone metabolic marker, a sex, a race, a menopause status, childbirth information, an age, a state of a cortical bone, a state of a cancellous bone, a state of a trabecular bone of the cancellous bone, disease information, bone assessment information, medication information, a presence or absence of a fracture, the number of fractures, a location of the fracture, and a history of fractures. More specifically, for example, at least one selected from the group consisting of a bone formation marker, a bone resorption marker, a bone quality marker (for example, a vitamin K value), a cortical bone thickness, a trabecular bone density, a trabecular bone direction, and a trabecular bone score can be employed for the bone quality, but the bone quality is not limited thereto. The disease information may include, for example, at least one selected from the group consisting of osteoporosis, rheumatism, osteonecrosis (such as femur head necrosis), systemic sclerosis, renal disease, osteopetrosis, and the like. The bone assessment information may include information assessed by using Fracture Risk Assessment Tool (FRAX (registered trademark)). The medication information may include, for example, at least one selected from the group consisting of a trade name, a common name, a dosage, an administration period, and an administration method (for example, oral, intravenous infusion, intramuscular infusion, or subcutaneous infusion) of a medication including at least one selected from the group consisting of a bone-resorption-suppressing medication, a bone-formation-promoting medication, and other medications (for example, a calcium preparation, a vitamin preparation, or a female hormone preparation).

The bone quality may also include, for example, a type of medullary cavity shape. For example, Dorr classification can be employed for the medullary cavity shape. The medullary cavity shape can be classified as follows using, for example, the thickness of the cortical bone and/or the shape of the medullary cavity.
- Type A: a type in which the cortical bone is thick and the medullary cavity is narrow and thin.
- Type B: a type that is intermediate between Type A and Type C and has neither a narrow nor wide medullary cavity.
- Type C: a type in which the cortical bone is thin and the medullary cavity is widened.

The first learning model is trained using first training data including a second image showing a bone of a person and bone information regarding at least any of a bone density, a bone mass, and a bone quality of the bone. The second image in the first training data may include an image showing at least a part of the bone in which the implant is placed. Here, the bone of the person may be a bone that includes bones in the same site as the target bone, or may be a bone that does not include bones in the same site as the target bone. The second image may also be, for example, an image showing at least a region that includes the entire bone in which the implant is placed. The second image may be, for example, an image showing a region that includes at least a part of the implant and a part of the bone surrounding the implant.

In the present disclosure, an implant can refer to an artificial object that is placed in the human body by surgical (including dental) operation. The implant is applicable to an implant placed in a target bone, and to an implant placed in each of the bones of a plurality of patients. The implants may include, for example, at least any of an artificial joint, a spinal implant, a trauma implant, a plastic implant, and a dental implant. The artificial joint may be, for example, at least any of an artificial hip joint, an artificial knee joint, an artificial shoulder joint, an artificial elbow joint, an artificial ankle joint, and an artificial finger joint. The spinal implant may be, for example, at least any of an instrumentation, a cage, an artificial intervertebral disc, and an artificial vertebral body. The trauma implant may be at least any of a plate, a screw, and a nail. The plastic implant may be a skull plate and/or a nasal bone prosthesis. The implant may also include, for example, bone cement to fix the implant to the bone. As the bone cement, for example, a material containing polymethyl methacrylate as a main component can be used.

### Configuration of Information Processing System 100a

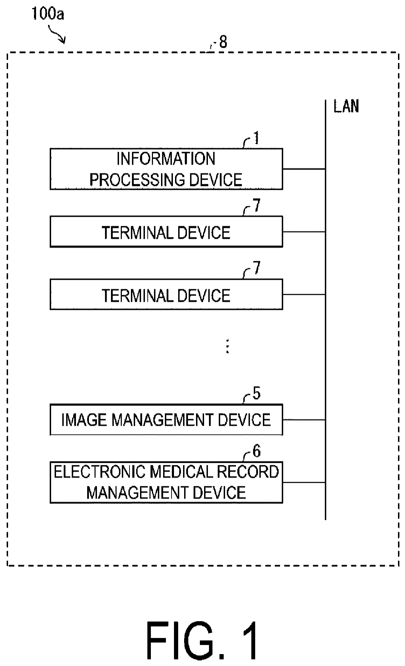

First, a configuration of an information processing system 100a according to one aspect of the present disclosure will be described with reference to FIG. 1. FIG. 1 is a diagram illustrating a configuration example of the information processing system 100a in a medical facility 8 in which the information processing device 1 is introduced.

The information processing system 100a includes the information processing device 1 and one or more terminal devices 7 communicatively connected to the information processing device 1. The information processing device 1 estimates first estimation information regarding at least any of bone information of a bone density, a bone mass, and a bone quality of a target bone, from a first image showing at least a part of the target bone. The information processing system 100a is a computer that transmits the first estimation information to the terminal device 7. The first estimation information can be useful information for a doctor or the like to detect signs of risks that occur in the target bone and an implant placed in the target bone. Therefore, the information processing device 1 according to one embodiment of the present disclosure is a device that outputs information that is useful for a doctor or the like to estimate the risks that occur in the prognosis of a subject to whom an implant placement surgery has been applied.

The terminal device 7 functions as an outputter in the information processing system 100a, and outputs information received from the information processing device 1. The terminal device 7 is, for example, a computer used by medical personnel such as a doctor (medical personnel) belonging to the medical facility 8. The terminal device 7 may be installed, for example, in the medical facility 8 or in a company that provides analysis services, or may be in a cloud. When the image management device 5 is a cloud, the first image and/or the second image can be acquired via a communication network. The terminal device 7 may be, for example, a device having a function of outputting, on a paper medium, information received from the information processing device 1. The terminal device 7 is, for example, a personal computer, a tablet terminal, or a smartphone. The terminal device 7 includes a communicator that transmits and receives data to and from another device, an inputter such as a keyboard and a microphone, a display capable of displaying information transmitted from the information processing device 1, an outputter such as a speaker, and the like.

An example is illustrated in which a local area network (LAN) is disposed and the information processing device 1 and the terminal devices 7 are connected to the LAN in the medical facility 8 illustrated in FIG. 1, but the present disclosure is not limited thereto. For example, the Internet, a telephone communication line network, an optical fiber communication network, a cable communication network, a satellite communication network, or the like may be applied to the network in the medical facility 8. The information processing system 100a can be adapted to a hospital information system (HIS), a radiology information system (RIS), a picture archiving and communication system (PACS), or the like in the medical facility 8. Communication in the information processing system 100a conforms to an international standard such as Digital Imaging and Communications in Medicine (DICOM).

In addition to the information processing device 1 and the terminal device 7, an image management device 5 and an electronic medical record management device 6 may be communicatively connected to the LAN in the medical facility 8. The image management device 5 and the electronic medical record management device 6 may be installed in the medical facility 8 or in a facility outside the medical facility 8. The image management device 5 is a computer functioning as a server for managing images captured in the medical facility 8. In this case, the information processing device 1 may acquire a first image showing at least a part of the target bone from the image management device 5. The information processing device 1 may acquire a first image showing at least a part of the target bone from an image management device 5 and an electronic medical record management device 6 installed in a facility outside the medical facility 8. The electronic medical record management device 6 is a computer functioning as a server for managing electronic medical record information of a subject who has undergone a medical examination in the medical facility 8. The electronic medical record information may include attribute information of the subject and surgical information including information regarding the surgical procedure for the implant placement surgery applied to the target bone.

The LAN in the medical facility 8 may be communicatively connected to an external communication network. In the medical facility 8, the information processing device 1 and the terminal device 7 may be directly connected to each other without going through the LAN.

### Configuration of Information Processing Device 1

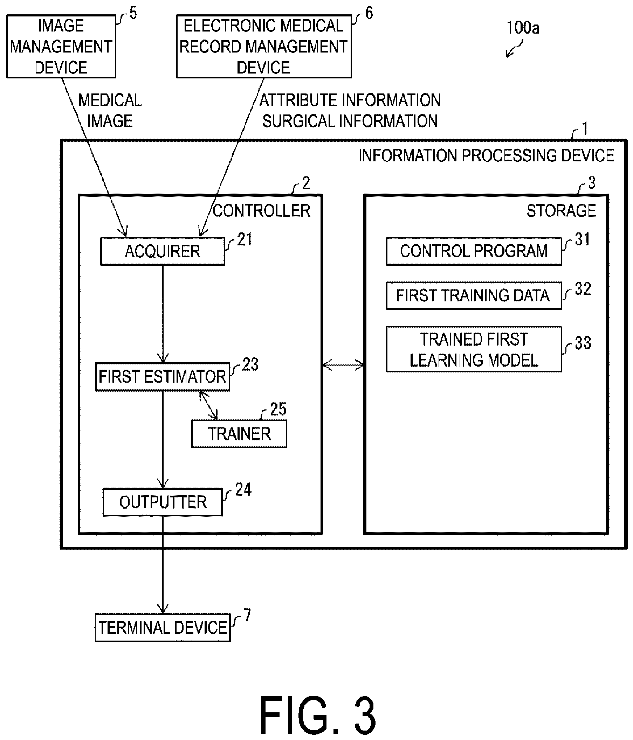

A configuration of the information processing device 1 applied to the information processing system 100a illustrated in FIG. 1 is described with reference to FIG. 3. FIG. 3 is a block diagram illustrating an example of a configuration example of the information processing device 1.

The information processing device 1 includes a controller 2 that integrally controls each component of the information processing device 1, and a storage 3 that stores various types of data to be used by the controller 2. The controller 2 includes an acquirer 21, a first estimator 23, an outputter 24, and a trainer 25. The storage 3 stores a control program 31, which is a program for performing various types of controls of the information processing device 1, as well as first training data 32 and a trained first learning model 33.

### Acquirer 21

The acquirer 21 acquires input information including a first image showing at least a part of a target bone. For example, the acquirer 21 illustrated in FIG. 3 may be capable of acquiring the first image from the image management device 5. The input information is input data input to the first estimator 23. The acquirer 21 may acquire the attribute information of the subject and the surgical information from the electronic medical record management device 6 in the medical facility 8.

### First Estimator 23

The first estimator 23 estimates the above-mentioned first estimation information by inputting input information into the trained first learning model 33. Here, the trained first learning model 33 is trained in advance using the first training data 32. The first training data may be data including a second image showing a bone of a person and bone information regarding at least any of a bone density, a bone mass, and a bone quality of the bone. For example, the first training data may be data including at least a second image showing a bone in which no implant is placed, and bone information regarding at least any of a bone density, a bone mass, and a bone quality of the bone, for each of a plurality of patients. Alternatively, the first training data may be data including the second image and bone information regarding at least any of a bone density, a bone mass, and a bone quality of the bone in which the implant is placed, for each of a plurality of patients.

The first estimator 23 may estimate first estimation information including bone information regarding at least any of a bone density, a bone mass, and a bone quality of each of a plurality of sites in the target bone, including a site proximate to the implant.

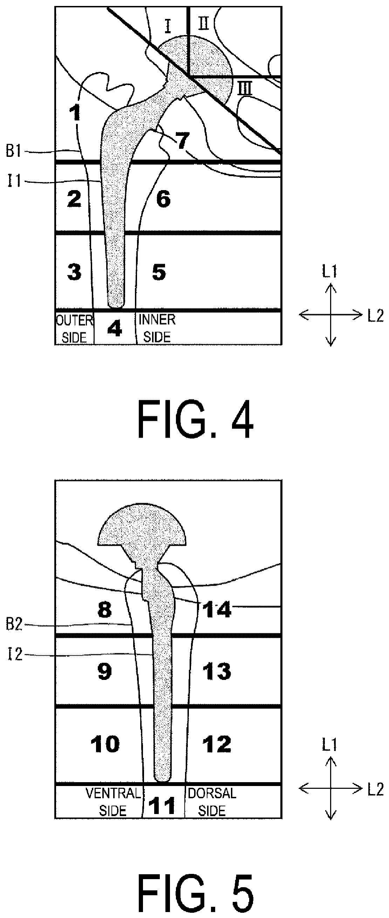

The plurality of sites can be set at any site proximate to the implant. Any site proximate to the implant may be a region shown in one image. For example, any site proximate to an implant may refer to a site that contacts the implant. Any site proximate to the implant may also refer to a site that does not contact the implant. The plurality of sites will be described using the stem of a cementless artificial hip joint as an example, but the present disclosure is not limited thereto. Each of the plurality of sites may be sites arranged along a first direction L1 from an insertion port side (proximal side) into which the stem of the implant is inserted to a tip side (distal side) of the stem. As the plurality of sites, for example, regions divided by the Gruen classification can be used as illustrated in FIG. 4. FIG. 4 is a view illustrating an example of a plurality of sites in a target bone (right femur) B1. FIG. 4 is a view of an artificial hip joint (that is, an implant I1) placed in the right hip joint as viewed from the ventral side (anterior). That is, in FIG. 4, the left side is the outer side (right arm side) and the right side is the inner side (left arm side). FIG. 4 illustrates a site 1 (outer side) and a site 7 (inner side), which are located closest to the insertion port, a site 2 (outer side) and a site 6 (inner side), which are next closest to the insertion port, a site 3 (outer side) and a site 5 (inner side), which are next closest to the insertion port, and a site 4, which is the furthest from the insertion port.

When the implant placed in the subject's bone is an artificial hip joint implant including a stem, the target bone is the femur in which the stem is placed. In this case, the first estimator 23 may estimate a plurality of pieces of first estimation information within a region of the femur, which is the target bone, classified by the Gruen classification. Here, the plurality of pieces of first estimation information may include first estimation information within a proximal region of the femur in which the stem is placed.

For example, the first estimator 23 may estimate bone information regarding at least one selected from the group consisting of a bone density, a bone mass, and a bone quality for each of the plurality of sites thus divided. In this case, the first estimator 23 may estimate different items for each of the plurality of sites, or may estimate the same item. For example, the first estimator 23 may estimate the bone density, the bone mass, and the bone quality (for example, trabecular bone score) for the sites 1, 7, 2, and 6, and may estimate only the bone density for the sites 3, 4, and 5. Alternatively, the first estimator 23 may estimate only the bone density, for example, for all of the sites 1 to 7. A bone density may be a value related to the density of a bone. A bone density may be represented by at least one type of bone mineral density per unit area (g/cm²), bone mineral density per unit volume (g/cm³), YAM (%), AGE, T-score, and Z-score. YAM (%) is an abbreviation for "Young Adult Mean" and is sometimes called a young adult average percentage. For example, the bone density of the bone may be a value expressed as bone mineral density per unit area (g/cm²) and YAM (%). AGE may be a value relative to an average value of the same age or the same age group. The bone density of the bone may be an index determined by a guideline or may be a unique index. The bone density can be, for example, a value used in osteoporosis guidelines (such as, but not limited to, the 2015 edition of Prevention and Treatment Guidelines of the Japan Osteoporosis Society, General Incorporated Association).

The first estimator 23 may estimate the first estimation information for all of the sites 1 to 7 as the first estimation information. Alternatively, the first estimator 23 may estimate the first estimation information for only a part of the sites as the first estimation information. More specifically, in order to estimate the loosening of the implant in the target bone, bone information including at least one selected from the group consisting of the bone density, the bone mass, and the bone quality at the site of the target bone B1 on the insertion port side where the stem of the implant I1 is inserted (that is, the sites 1, 2, 6, and 7 in FIG. 4) is more important. Therefore, the first estimator 23 may estimate the first estimation information, for example, for only the sites 1 and 7 as the first estimation information. Alternatively, the first estimator 23 may estimate the first estimation information with different estimation accuracy for each site based on the importance of the site with respect to loosening of the implant I1. Estimating with different accuracy for each site includes, for example, changing the number of calculations for each site, changing the learning model for each site, changing the variables used during calculation for each site, and the like. For example, the sites 1 and 7 may be estimated with high accuracy, and the other sites may be estimated with lower accuracy than the sites 1 and 7.

Alternatively, each of the plurality of sites may be a site along a second direction L2 from the medullary cavity side to the outer shell side of the target bone B1 (that is, a direction away from the axis of the stem of the placed implant I1). In order to estimate the loosening of the implant in the target bone B1, bone information including at least one selected from the group consisting of the bone density, the bone mass, and the bone quality in the site of the target bone B1 near the stem of the implant I1 on the medullary cavity side of the target bone B1 is more important.

The plurality of sites may be set two-dimensionally by dividing the region in each of the first direction L1 and the second direction L2. The plurality of sites may be set in regions having different areas. The plurality of sites may be set in regions where a specific portion has a smaller area. The plurality of sites may be freely set as regions depending on attribute information of the subject, for example. For example, when the subject has a history of fractures, the area of the specific portion may be set to be smaller than that of a subject without a history of fractures. More specifically, the area of the site may be set to be smaller toward the sites 1 and 7 in the first direction L1 than the other sites. In the second direction L2, the area of the site closer to the implant I1 may be set to be smaller than the area of the site further away from the implant. Furthermore, in a case where cement is used when placing the artificial hip joint, the plurality of sites may be set, for example, so as to avoid the cement portion by considering the cement as a part of the artificial joint.

### Example of Plurality of Sites in Implant

Although the stem of a cementless artificial hip joint has been described above, other artificial joints can also be similarly divided into regions and the first estimation information can be estimated for each region, as shown in Examples 1 to 3 below.

Example 1: In the case of a cementless artificial hip joint, the plurality of sites can be set at any site proximate to the cup. The cup is placed, for example, in the acetabulum of the pelvis. For the plurality of sites of the acetabulum proximate to the cup, for example, regions I to III divided by the Chanley classification can be used, as illustrated in FIG. 4.

When the implant placed in the subject's bone is an artificial hip joint implant including a cup, the target bone is the acetabulum in which the cup is placed. In this case, the first estimator 23 may estimate a plurality of pieces of first estimation information within a region of the acetabulum, which is the target bone, classified by the Charnley classification.

Example 2: The regions divided by the Gruen classification are not limited to the example illustrated in FIG. 4, but may also be applied when the artificial hip joint is viewed from the side. FIG. 5 is a view illustrating an example of a plurality of sites in a target bone (left femur) B2. FIG. 5 is a view of an artificial hip joint (that is, an implant I2) placed in the left hip joint as viewed from the left arm side (lateral side). That is, in FIG. 5, the left side is the ventral side and the right side is the dorsal side. FIG. 5 illustrates a site 8 (ventral side) and a site 14 (dorsal side), which are located closest to the insertion port, a site 9 (ventral side) and a site 13 (dorsal side), which are next closest to the insertion port, a site 10 (ventral side) and a site 12 (dorsal side), which are next closest to the insertion port, and a site 11, which is the furthest from the insertion port.

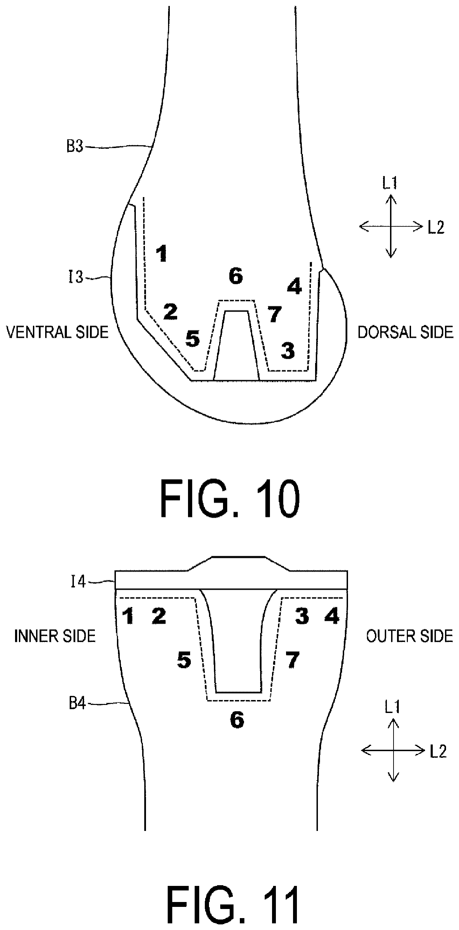

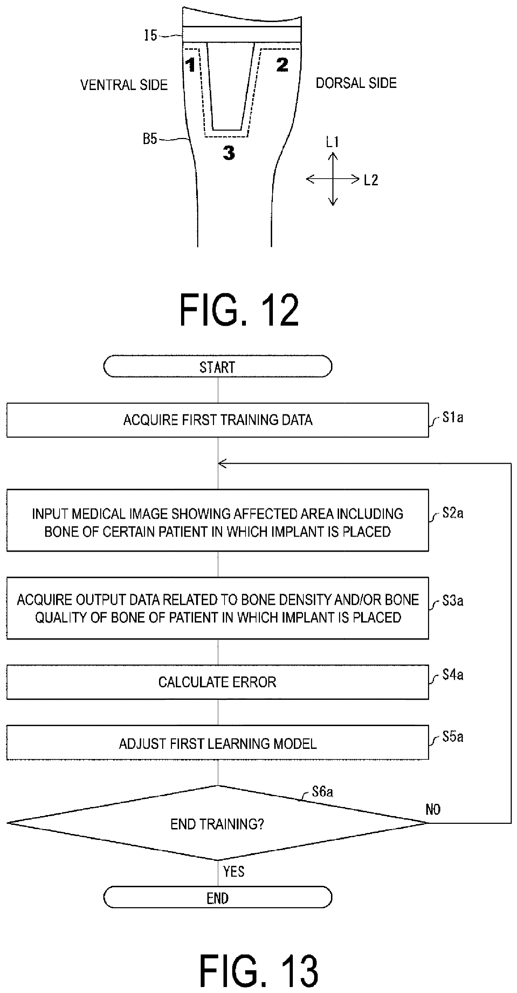

Example 3: The plurality of sites that can be set in the case of an artificial knee joint will be described with reference to FIGs. 10 to 12. FIG. 10 is a view illustrating an example of a plurality of sites in a target bone (left femur) B3. FIG. 10 is a view of an artificial knee joint (that is, an implant I3) placed in the left femur B3 as viewed from the left arm side (lateral side). That is, in FIG. 10, the left side is the ventral side and the right side is the dorsal side. In FIG. 10, sites 1 to 7 are illustrated. FIG. 11 is a view illustrating an example of a plurality of sites in a target bone (left tibia) B4. FIG. 11 is a view of an artificial knee joint (that is, an implant I4) placed in the left tibia B4 as viewed from the ventral side (anterior). That is, in FIG. 11, the left side is the inner side (right arm side) and the right side is the outer side (left arm side). In FIG. 11, sites 1 to 7 are illustrated. FIG. 12 is a view illustrating an example of a plurality of sites in a target bone (left tibia) B5. FIG. 12 is a view of an artificial knee joint placed in the left tibia B5 as viewed from the left arm side (anterior). That is, in FIG. 12, the left side is the ventral side and the right side is the dorsal side. In FIG. 12, sites 1 to 3 are illustrated.

In any of Examples 1 to 3, each of the plurality of sites may be sites arranged along a first direction L1 from an insertion port side (proximal side) into which the stem of the implants I2 to I5 is inserted to a tip side (distal side) of the stem. Each of the plurality of sites may be a site along a second direction L2 from the medullary cavity side to the outer shell side of the target bone (that is, a direction away from the axis of the stem of the placed implants I2 to I5).

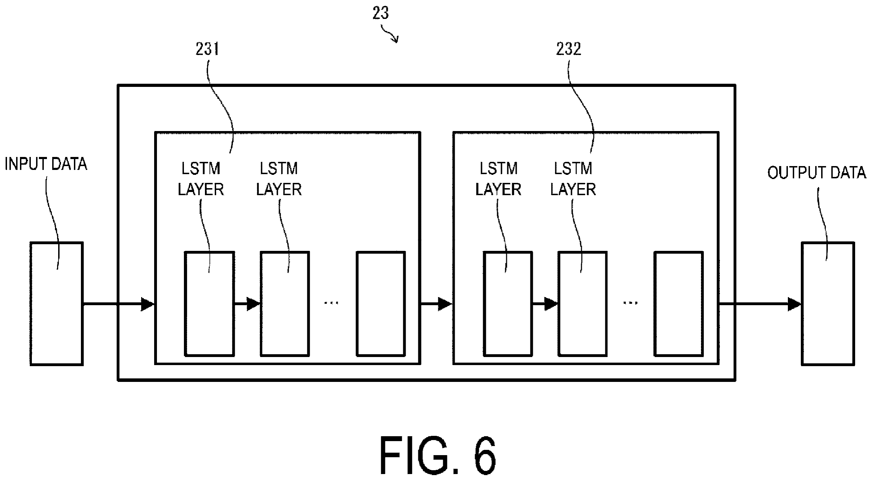

In response to the input information being input to an input layer 231 (see FIG. 6), the first estimator 23 performs an operation, based on the trained first learning model, and outputs, from an output layer 232, a surgical treatment method suitable for the affected bone of the subject and an implant usable in the surgical treatment method (see FIG. 6). As an example, the first estimator 23 may extract a feature from the input information and use the feature as input data. In extracting the feature, known algorithms as listed below may be applied.
- Convolutional neural network (CNN)
- Autoencoder
- Recurrent neural network (RNN)
- Long short-term memory (LSTM)
- Convolutional long short-term memory (ConvLSTM).

The trained first learning model 33 is an operation model used by the first estimator 23 in performing an operation, based on the input data. The trained first learning model 33 is generated by the trainer 25 executing machine learning using the first training data 32, which will be described below, on an untrained neural network. Here, the trained first learning model 33 may also be applied to an animal other than a human. In such a case, the "patient" in the first training data 32 may be a biological species of the same type as the "subject." In other words, the information processing device 1 according to the present disclosure is also capable of estimating first estimation information regarding at least any of bone information of a bone density, a bone mass, and a bone quality of a bone in which an implant of an animal other than a human is placed. A specific example of the first training data 32, a configuration of the neural network, and the training processing will be described below.

### Outputter 24

The outputter 24 transmits first estimation information estimated by the first estimator 23 to the terminal device 7. The information processing device 1 may include a display (not illustrated). In such a case, the outputter 24 displays each piece of information described above on the display. The display may, for example, change the color of each of the divided sites according to the received first estimation information. The display may display, for example, a heat map according to the received first estimation information.

### Trainer 25

The trainer 25 controls training processing for the untrained neural network. The trainer 25 creates a trained neural network (trained first learning model 33) functioning as the first estimator 23 by executing training processing on an untrained neural network. The first training data 32 (described below) is used for such training. A specific example of training executed by the trainer 25 will be described below.

### Configuration of First Estimator 23

A configuration of the first estimator 23 will be described below with reference to FIG. 6. The configuration illustrated in FIG. 6 is merely an example and the configuration of the first estimator 23 is not limited thereto.

As illustrated in FIG. 6, the first estimator 23 performs operations based on the trained first learning model 33 on the input data input to the input layer 231 to output output data from the output layer 232. The output data is bone information regarding at least any of the bone density, the bone mass, and the bone quality of the bone in which the implant is placed.

The first estimator 23 in FIG. 6 includes a neural network including the input layer 231 and the output layer 232. FIG. 6 illustrates a case where the neural network is an LSTM; however, the neural network is not limited thereto. For example, the neural network may be a ConvLSTM network in which CNN and LSTM are combined. The input layer 231 can extract a feature for a change in the input data. The output layer 232 can calculate a new feature, based on the feature extracted in the input layer 231 and the temporal change and the initial value of the input data. The temporal change is a difference in time between a point in time at which the input data is acquired and a point in time to be estimated, and may be input by the person using the system as one year, three years, five years, ten years, 20 years, or 50 years, or a difference that is automatically determined within the system may be input. The initial value may be the value of the bone mass or the bone quality at the point in time at which the input data is acquired. The initial value may be estimated by the first estimator 23, or a value of a bone mass or a bone quality measured by a separate device may be used. Each of the input layer 231 and the output layer 232 includes a plurality of LSTM layers. Each of the input layer 231 and the output layer 232 may include three or more LSTM layers.

### Training Processing by Trainer 25

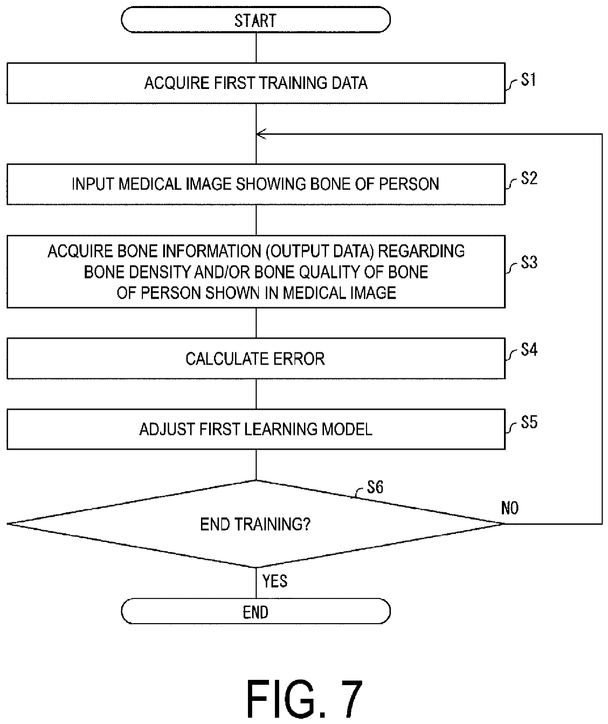

Training processing for generating the trained first learning model 33 will be described below with reference to FIG. 7. FIG. 7 is a flowchart illustrating an example of a flow of training processing by the trainer 25.

The trainer 25 acquires the first training data 32 from the storage 3 (step S1). The first training data 32 includes an explanatory variable and a response variable to be input to the input layer 231. Here, the explanatory variable is a second image showing a bone of a person, and the response variable may be bone information regarding at least any of the bone density, the bone mass, and the bone quality of the bone of the person shown in the second image. In addition to the bone information, the response variables may include a region and/or position information of the bone information, or may include information regarding loosening of the implant. A region or position information of the bone information may be used as an explanatory variable. The position information may be any information indicating a position on an image, and may be XY coordinates on the image, for example. Alternatively, the position information may be information indicating the interface between the bone and the implant, or a position a predetermined distance away from the interface of the implant. Alternatively, the position information may be the name of a particular site of the human body. The position information may be, for example, the trochanter for the proximal femur, the posterior condyle for the distal femur, or the medial side for the proximal tibia. The information regarding the loosening of the implant may be at least one selected from the group consisting of the following: the presence or absence of loosening, the probability of loosening occurring, the presence or absence of a bone radiograph, the thickness of the bone radiograph, the range of the bone radiograph, changes in the thickness or range of the bone radiograph, sensation or pain felt by the patient, and changes in sensation or pain felt by the patient.

Next, the trainer 25 inputs a second image showing a bone of a certain person to the input layer 231 (step S2).

Next, the trainer 25 acquires bone information (that is, output data) regarding at least any of the bone density, the bone mass, and the bone quality of the bone of the person shown in the second image input in step S2 from the output layer 232 (step S3). The output data contains the same contents as the response variable of the first training data 32. In FIG. 7, the order of step S2 and step S3 may be reversed. Alternatively, in FIG. 7, step S2 and step S3 may be executed simultaneously.

Next, the trainer 25 acquires a response variable related to the person shown in the second image input in step S2, which is included in the first training data 32. The trainer 25 compares the output data acquired in step S3 with the response variable for the person, calculates an error (step S4), and adjusts the first learning model 33 being trained so as to reduce the error (step S5).

Any known method is applicable to the adjustment of the first learning model being trained. For example, backpropagation may be employed as a method for adjusting the first learning model. The adjusted first learning model becomes a new first learning model, and in subsequent operations, the first estimator 23 uses the new first learning model. In an adjustment stage of the first learning model, the parameters (for example, a filter coefficient and a weighting factor) used in the first estimator 23 may be adjusted.

If the error is not within a predetermined range, and if explanatory variables for all the persons contained in the first training data 32 are not input (NO in step S6), the trainer 25 returns to step S2 and repeats the training processing. If the error is within the predetermined range, and if the explanatory variables for all the persons contained in the first training data 32 are input (YES in step S6), the trainer 25 ends the training processing.

When the above-described training processing is employed, the first estimator 23 can estimate first estimation information regarding at least any of the bone information of the bone density, the bone mass, and the bone quality of a part of a target bone from input information including a first image showing at least a part of the target bone in which an implant is placed.

### Another Example of Training Processing by Trainer 25

Another example of training processing for generating the trained first learning model 33 will be described below with reference to FIG. 13. FIG. 13 is a flowchart illustrating another example of a flow of training processing by the trainer 25.

The trainer 25 acquires the first training data 32 from the storage 3 (step S1a). The first training data 32 includes an explanatory variable and a response variable to be input to the input layer 231. Here, the explanatory variable includes a second image showing at least a bone in which an implant is placed for each of the plurality of patients, and the response variable is bone information regarding at least any of a bone density, a bone mass, and a bone quality of the bone in which the implant is placed.

Next, the trainer 25 inputs a second image showing at least a part of a bone of a certain patient in which an implant is placed (referred to as a patient A) to the input layer 231 (step S2a).

Next, the trainer 25 acquires output data related to at least any of bone information of a bone density, a bone mass, and a bone quality of the bone of the patient A in which the implant is placed from the output layer 232 (step S3a). The output data contains the same contents as the response variable of the first training data 32. In FIG. 13, the order of step S2a and step S3a may be reversed. Alternatively, in FIG. 13, step S2a and step S3a may be executed simultaneously.

Next, the trainer 25 acquires a response variable for the patient A included in the first training data 32. The trainer 25 compares the output data acquired in step S3a with the response variable for the patient A, calculates an error (step S4a), and adjusts the first learning model 33 being trained so as to reduce the error (step S5a).

Any known method is applicable to the adjustment of the first learning model being trained. For example, backpropagation may be employed as a method for adjusting the first learning model. The adjusted first learning model becomes a new first learning model, and in subsequent operations, the first estimator 23 uses the new first learning model. In an adjustment stage of the first learning model, the parameters (for example, a filter coefficient and a weighting factor) used in the first estimator 23 may be adjusted.

If the error is not within a predetermined range, and if explanatory variables for all the patients contained in the first training data 32 are not input (NO in step S6a), the trainer 25 returns to step S2a and repeats the training processing. If the error is within the predetermined range, and if the explanatory variables for all the patients contained in the first training data 32 are input (YES in step S6a), the trainer 25 ends the training processing.

When the above-described training processing is employed, the first estimator 23 can estimate first estimation information regarding at least any of the bone information of the bone density, the bone mass, and the bone quality of a part of a target bone from input information including a first image showing at least a part of the target bone in which an implant is placed.

### First Aspect of First Embodiment

The first training data 32 may further include attribute information for each of the plurality of patients and/or surgical information regarding the placement surgery used to place the implant in the bone of each patient. By using such first training data 32, the trained first learning model 33 can more accurately estimate first estimation information regarding at least any of bone information of a bone density, a bone mass, and a bone quality of a target bone, from the input information. In this case, the input information may include at least a first image showing the target bone, attribute information of the subject, and surgical information regarding the placement surgery used to place the implant in the target bone.

In the first training data 32 (and the input information), the attribute information may include at least any of the following for each patient (and subject): an age, a sex, a height, a weight, a race, a menopause status, a presence or absence of a fracture, the number of fractures, a location of the fracture, a history of fractures, information regarding lifestyle habits, information regarding medications being taken (medication information), information indicating the results of blood tests (blood test information), urine test information, saliva test information, a medical history, a medical history of the subject's family members, genetic information, childbirth information, and menopause information, a menopause prediction based on hormone information, and childbirth information. In the first training data 32 (and the input information), the surgical information may include at least any of the following: a model of an implant placed in each patient's bone (and the target bone), a size of the implant, a surgical procedure for a placement surgery applied, a surgical time for the placement surgery, the amount of bleeding during the placement surgery, information indicating a medical facility in which the placement surgery has been performed, and information indicating a surgeon for the placement surgery. The lifestyle habit may be, for example, a sleep time, a wake-up time, a sleep time, a daily exercise amount, a meal content, a meal time, a meal time period, a blood glucose level, and the like. The meal content includes, for example, at least one selected from the group consisting of a meal name, an ingested food material, and an intake amount. The meal content may be an estimated intake including at least one selected from the group consisting of, for example, calcium, vitamin B, vitamin D, and vitamin K. For example, a designated value estimated from a parameter acquired by a wearable device may be employed for the blood glucose level. The information processing device 1 may acquire the attribute information of the subject from the attribute information management device 4. When the attribute information is added to the acquired first image and/or second image, the information processing device 1 may extract the attribute information from the first image and/or the second image.

The medication information may include information such as a name of a medication, a taken amount of the medication, and a period of taking the medication. The information on the medication being taken may include information on a steroid agent being used. The blood test information may be information on the result of at least any of, for example, a biochemical test, a glucose metabolism test, and an endocrine test.

When applying the implant placement surgery, both the attribute information and the surgical information of the patient (and the subject) may be information regarding the prognosis of the affected area to which the implant placement surgery has been applied, or information that may affect the prognosis of the affected area. According to this configuration, the information processing device 1 can provide the first estimation information to a doctor or the like with higher accuracy. Thus, the information processing device 1 can support estimating the risk that occurs in the prognosis of the target bone.

### Second Aspect of First Embodiment

The adjustment of the trained first learning model 33 may be executed in a computer other than the information processing device 1. In such a case, the trained first learning model 33 may be installed in and then used by the information processing device 1. That is, in the information processing device 1, the trainer 25 is not an essential component.

### Processing Performed by Information Processing Device 1

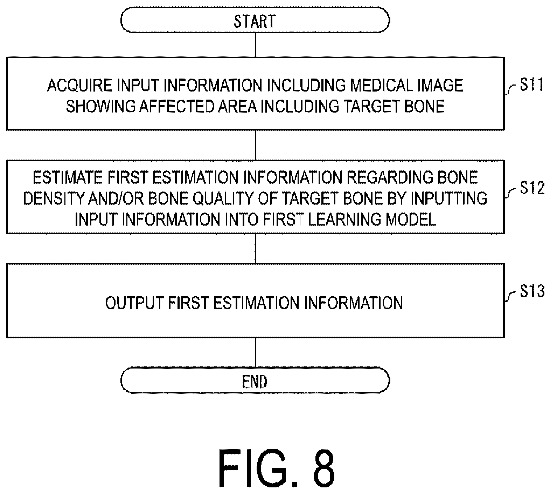

A flow of processing performed by the information processing device 1 will be described below with reference to FIG. 8. FIG. 8 is a flowchart illustrating an example of a flow of processing performed by the information processing device 1. FIG. 8 illustrates an example of processing performed by the information processing device 1 when the information processing device 1 outputs first estimation information regarding bone information of a target bone from input information.

First, the acquirer 21 acquires input information including a first image showing at least a part of a target bone (step S11: acquiring step).

Next, the first estimator 23 estimates first estimation information regarding at least any of bone information of a bone density, a bone mass, and a bone quality of the target bone, by inputting the input information into the trained first learning model 33 (step S12: estimating step).

The outputter 24 outputs the first estimation information estimated in step S12 (step S13: outputting step).

According to this configuration, the information processing device 1 and the information processing system 100a can output useful information for a doctor or the like to estimate the risks that may occur in the affected area of a subject to whom an implant placement surgery has been applied.

### Second Embodiment

Another embodiment of the present disclosure will be described below. For convenience of description, members having the same functions as those of the members described in the above-described embodiment are denoted by the same reference signs, and description thereof is not repeated.

The information processing system 100a may output second estimation information regarding an event that may occur in the subject due to the placement of the implant, the second estimation information being estimated based on the input information.

### Configuration of Information Processing Device 1a

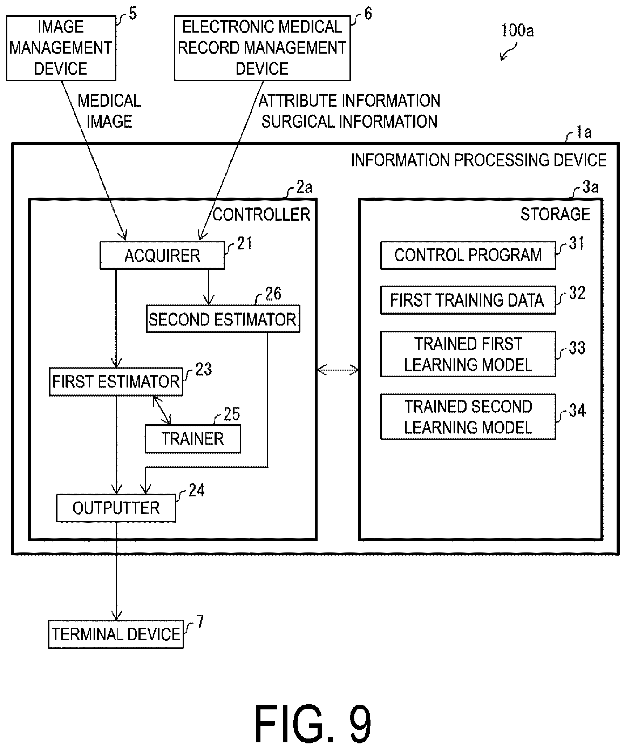

A configuration of an information processing device 1a having such a configuration will be described with reference to FIG. 9. FIG. 9 is a block diagram illustrating an example of a configuration example of the information processing device 1a. The information processing device 1a can be applied to the information processing system 100a and an information processing system 100b.

The information processing device 1a includes a controller 2a that integrally controls each component of the information processing device 1a, and a storage 3a that stores various types of data to be used by the controller 2a. The controller 2a includes a second estimator 26 in addition to the acquirer 21, the first estimator 23, the outputter 24, and the trainer 25. The storage 3a stores a trained second learning model 34 in addition to the control program 31, which is a program for performing various types of controls of the information processing device 1a, the first training data 32, and the trained first learning model 33.

The second estimator 26 estimates second estimation information from the first image included in the input information, using the second learning model 34 trained using the second training data. Here, the second training data is data including a third image showing a bone of a person and progress information regarding an event that has occurred due to the placement of an implant (third implant) in the bone. The second training data may be, for example, data including a third image showing at least a part of the bone in which the implant is placed, and progress information regarding an event that has occurred due to the placement of the implant, for each of a plurality of patients.

The outputter 24 transmits the estimated second estimation information to the terminal device 7. The third image included in the second training data may be, for example, an image captured until a certain period of time (for example, six months) elapses after the progress information after the implant is placed is acquired. The second training data may further include a combination of the following information (i) and (ii).
(i) Information regarding future bone density obtained from a prediction result regarding the bone state when a predetermined period (for example, five years) has elapsed since a third image was captured in the past (for example, at the time of implant placement). The future bone density may be information regarding the bone state or the bone density when a predetermined period has elapsed since the third image was captured in the past (for example, an actual measurement value of the bone density measured when a predetermined period has elapsed since the third image was captured in the past).
(ii) Information regarding the current (for example, the point in time at which the third image is captured) bone density of the bone. The current bone density of the bone may be an actual measurement value of the bone density measured when the third image is captured. Alternatively, the current bone density may be a bone density estimated from the analysis results of an image of the bone shown in the third image.

Here, the events that have occurred due to the placement of the implant may include at least any of the following.
- Loosening of a placed implant.
- Fracture of the bone in which the implant is placed.
- Dislocation of a joint associated with the bone in which the implant is placed.
- Infection of an affected area, including the bone in which the implant is placed.
- Fracture of the implant.

Loosening of a placed implant can be detected, for example, based on at least one selected from the group consisting of an X-ray image showing the implant and the bone interface in which the implant is placed, a bone radiograph, and a medical examination (for example, a medical interview and/or palpation, etc.) by a doctor. The loosening of a placed implant may be, for example, at least one selected from the group consisting of the sensation or pain felt by the patient, the interval between the implant and the bone (for example, the width of the gap), and the change in position of the implant from when it was placed, and the presence or absence of loosening and/or the degree of loosening. A fracture of a bone in which the implant is placed, dislocation of a joint associated with the bone in which the implant is placed, infection of an affected area including the bone in which the implant is placed, and a fracture of the implant may be detected, for example, based on an X-ray image of the affected area and/or a medical examination (for example, a medical interview and/or palpation, etc.) by a doctor. A fracture of a bone in which the implant is placed includes, for example, a fracture of a bone in the skeleton located around the bone in which the implant is placed.

The second training data may be, for example, progress information at one point in time, or may include progress information at a plurality of different points in time. When progress information at a plurality of different points in time is held as the second training data, the trend between the plurality of different points in time can be used as training data. For example, the trained second learning model 34 may be a learning model trained using second training data that includes, as explanatory variables, third images and progress information of the patient acquired at a plurality of different points in time, and as response variables, events that occurred due to the placement of the implant of the patient which is confirmed at the plurality of different points in time. The progress information may include, for example, information regarding at least one selected from the group consisting of the position of the bone radiograph, the thickness of the bone radiograph, the range of the bone radiograph, sensation or pain (for example, pain intensity, type of pain, and duration of pain) felt by the patient, the interval or angle between the implant and the bone, and the change in position of the implant from when it was placed to the current point in time.

By checking the second estimation information regarding the affected area in addition to the first estimation information regarding the affected area of the subject, a doctor or the like can accurately determine the risk that may occur in the affected area of the subject. Thus, the information processing device 1a can support a doctor or the like in estimating the risk that may occur in the prognosis of the affected area of the subject.

When loosening of an implant is predicted in this manner, the extent to which it will improve with treatment may also be indicated. It may be possible to predict how loosening of an implant will change as a result of administering treatment to a patient.

Loosening of an implant occurs due to a decrease in bone density of the bone around the placed implant. In other words, improvement in the bone density of the bone around the placed implant leads to improvement in loosening of the implant. A doctor or the like can refer to the first estimation information and the second estimation information to determine a treatment plan that includes prescribing a medication to a subject that is expected to have the effect of suppressing the occurrence of loosening of an implant and delaying the occurrence of loosening of an implant. Medications considered for prescription to a subject may include, for example, a medication having an effect on bone formation and a medication having an effect on bone resorption. Examples of the medication having an effect on bone formation may include, but are not limited to, an activated vitamin D3 preparation (for example, Calcitriol, Eldecalcitol, or Alfacalcidol), Teriparatide Acetate, Teriparatide (recombinant), and the like. Examples of the medication having an effect on bone resorption may include, but are not limited to, a calcitonin preparation, a bisphosphonate preparation, and an anti-RANKL monoclonal antibody.

### Third Embodiment

### Configuration of Information Processing System 100b

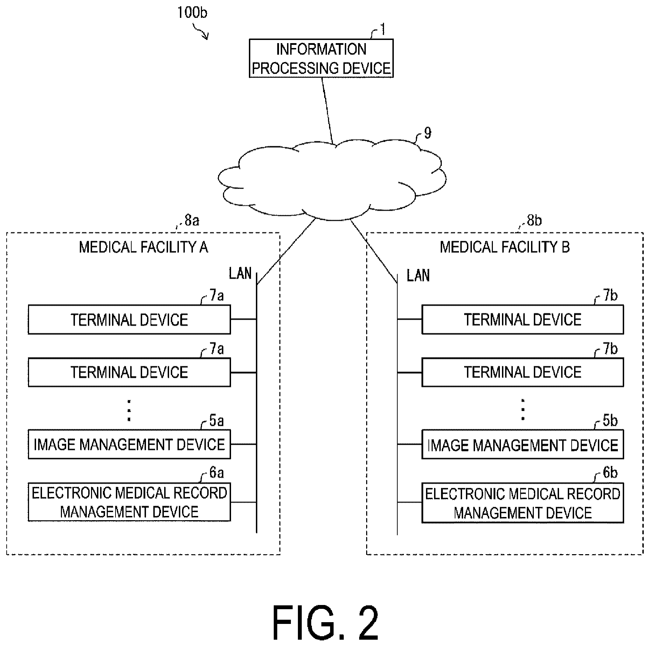

The information processing device 1 may be communicatively connected to LANs each disposed in the corresponding one of a plurality of the medical facilities 8 via a communication network 9, instead of a computer installed in the predetermined medical facility 8. FIG. 2 is a diagram illustrating a configuration example of the information processing system 100b according to another aspect of the present disclosure.

In addition to one or more terminal devices 7a, an image management device 5a and an electronic medical record management device 6a may be communicatively connected to the LAN in a medical facility 8a. In addition to a terminal device 7b, an image management device 5b and an electronic medical record management device 6b may be communicatively connected to the LAN in a medical facility 8b. In the present disclosure, when no particular distinction is made between the medical facilities 8a and 8b, each of the medical facilities 8a and 8b is referred to as a "medical facility 8." When no particular distinction is made between the terminal devices 7a and 7b, between the image management devices 5a and 5b, and between the electronic medical record management devices 6a and 6b, each of the terminal devices 7a and 7b, each of the image management devices 5a and 5b, and each of the electronic medical record management devices 6a and 6b are referred to as the "terminal device 7," the "image management device 5," and the "electronic medical record management device 6," respectively.

FIG. 2 illustrates an example in which the LANs in the medical facilities 8a and 8b are connected to the communication network 9. The information processing device 1 is not limited to the configuration illustrated in FIG. 2, as long as the information processing device 1 is communicatively connected to the image management device 5 and the electronic medical record management device 6 in each medical facility via the communication network 9. For example, the information processing device 1 may be installed in the medical facility 8a or in the medical facility 8b.

In the information processing system 100b employing such a configuration, the information processing device 1 can acquire a first image of a subject Pa examined in the medical facility 8a from the image management device 5a in the medical facility 8a. The information processing device 1 can acquire attribute information and surgical information of the subject Pa from the electronic medical record management device 6a in the medical facility 8a. The information processing device 1 transmits the first estimation information regarding the subject Pa to the terminal device 7a installed in the medical facility 8a. Similarly, the information processing device 1 can acquire attribute information and surgical information of a subject Pb from the electronic medical record management device 6b in the medical facility 8b. The information processing device 1 transmits the first estimation information regarding the subject Pb to the terminal device 7a installed in the medical facility 8b.

### Supplementary Note

The information processing device 1 need not include all the functional blocks included in the controller 2 of the information processing device 1. For example, in the information processing system 100a, the terminal device 7 may have the function of the acquirer 21, and the information processing device 1 may receive the input information from the terminal device 7. Similarly, in the information processing system 100b, the information processing device 1 may receive input information from the terminal device 7.

For example, in the information processing systems 100a and 100b, the function of the trainer 25 may be configured to install the trained first learning model 33, which has been trained by a computer other than the information processing device 1, into the information processing device 1.

For example, in the information processing systems 100a and 100b, the terminal device 7 or a computer other than the information processing device 1 may have the function of the second estimator 26. In the information processing systems 100a and 100b, the terminal device 7 or a computer other than the information processing device 1 may have the function of the second estimator 26.

### Fourth Embodiment

Another embodiment of the present disclosure will be described below. In an information processing device 1001 according to the present embodiment, third estimation information of bone information regarding the bone state of the subject is output. The third estimation information of bone information regarding the bone state of the subject is estimated using a third learning model 1034, based on input information including, as the first image and/or the second image in the first embodiment, a medical image showing a trabecular bone of a subject and/or a first virtual image that represents a color shading in the medical image for each predetermined area (pixel). The learning model in the present embodiment is trained using training data including information including a second medical image showing the trabecular bone of a predetermined person and trabecular bone information regarding the state of the trabecular bone of the predetermined person and/or a second virtual image that represents a color shading in the second medical image for each predetermined area.

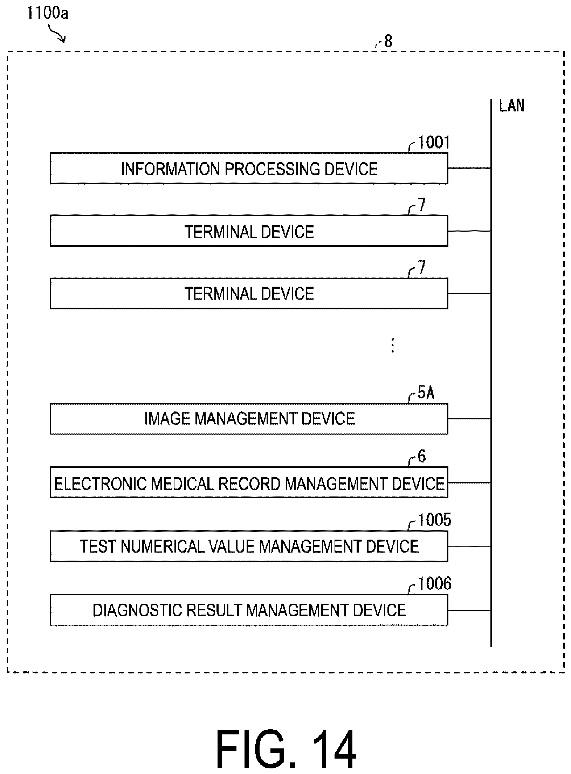

FIG. 14 is a diagram illustrating a configuration example of an information processing system 1100a in a medical facility 8 in which an information processing device 1001 is introduced. As illustrated in FIG. 14, the information processing system 1100a includes an information processing device 1001 instead of the information processing device 1 in the information processing system 100a according to the first embodiment. The information processing device 1001 estimates bone information regarding the bone state of the subject from medical information including a first image showing the trabecular bone of the subject and/or a first virtual image that represents a color shading in the first image for each predetermined area. The information processing device 1001 may estimate future bone information of the subject, or may estimate current bone information of the subject. The information processing device 1001 is a computer that transmits third estimation information of the bone information of the subject to the terminal device 7.

The information processing system 1100a includes an image management device 5A instead of the image management device 5 in the first embodiment. The information processing system 1100a may include a test numerical value management device 1005 and a diagnostic result management device 1006 in addition to the configuration of the information processing system 100a in the first embodiment. The information processing device 1001, the terminal device 7, the image management device 5A, the electronic medical record management device 6, the test numerical value management device 1005, and the diagnostic result management device 1006 may be communicatively connected via a LAN within the medical facility 8.

The image management device 5A generates a virtual image that represents the color shading in the first image for each predetermined area. The virtual image is generated from the shading of the cancellous bone and/or cortical bone portions and the portions other than the bones in the bone image information including the bone portions in the first image. More specifically, the virtual image can be generated as an image, for example, in which the bone shading from the bone image information is converted into a numerical value for each predetermined area, and the numerical value is represented as a color shading.

The predetermined area may be, for example, one pixel of the image as one unit, or a plurality of pixels of the image as one unit. In a predetermined area, at least one selected from the group consisting of the trabecular bone, the trabecular number (for example, the number of trabecular bones present per unit length), the trabecular spacing (for example, the spatial distance between trabecular bones), the trabecular width (for example, the width or the thickness of trabecular bones), the trabecular orientation (for example, the degree of alignment of the trabecular direction within the trabeculae), and the bone connectivity density (for example, the number of paths connecting the end portions of trabecular bones per unit area) may be considered. As the numerical value, for example, at least one selected from the group consisting of the bone mass (for example, a numerical value in g), the bone density, and the trabecular bone score can be used. As the color shading, for example, two-gradation colors consisting of white and black, grayscale (for example, two-gradation colors of white and black plus colors of the shading of gray represented by 254 intermediate gradations between white and black), or a heat map using a plurality of colors can be used. Note that the color shading is not limited to these, and for example, one gradation may be made by displaying white as transparent among two gradations, or a predetermined numerical value or more may be a target of shading. The information processing device 1 may acquire, from the image management device 5A, a medical image showing the trabecular bone of the subject, and a virtual image generated using the medical image.

The test numerical value management device 1005 is a computer functioning as a server for managing a test numerical value obtained by a test performed in the medical facility 8. The test numerical values include, for example, at least one selected from the group consisting of Kellgren-Lawrence (KL) classification, a bone morphology angle, a muscle mass, Mini Mental State Examination (MMSE), blood test numerical values (for example, at least one selected from the group consisting of a bone formation marker, a bone resorption marker, and a vitamin K value), a liver function marker, a uric acid value, bone assessment information, and a malignant tumor marker. The information processing device 1001 may acquire the test numerical value of the subject from the test numerical value management device 1005.

The diagnostic result management device 1006 is a computer functioning as a server for managing diagnostic results obtained by a diagnosis performed in the medical facility 8. The information processing device 1001 may acquire the diagnostic result of the subject from the diagnostic result management device 1006. The diagnostic result may include the presence or absence of a fracture and the progression of osteoporosis. For fractures, information regarding their cause, for example, fragility, stress or traumatic fractures may be added.

FIG. 14 illustrates an example of a configuration including the image management device 5A, the electronic medical record management device 6, the test numerical value management device 1005, and the diagnostic result management device 1006, but the present disclosure is not limited thereto. For example, the information processing system may include a management device having the functions of some or all of these devices.

### Configuration of Information Processing Device 1001

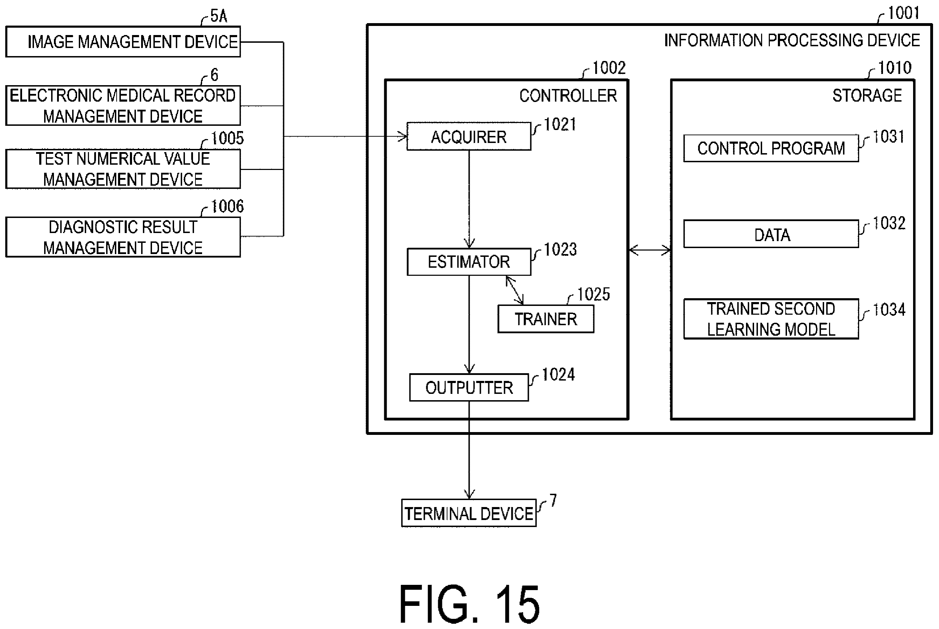

Next, a configuration of the information processing device 1001 applied to the information processing system 1100a illustrated in FIG. 14 is described with reference to FIG. 15. FIG. 15 is a block diagram illustrating an example of a configuration example of the information processing device 1001.

The information processing device 1001 includes a controller 1002 that integrally controls each component of the information processing device 1001, and a storage 1010 that stores various types of data to be used by the controller 1002. The controller 1002 includes an acquirer 1021, an estimator 1023, an outputter 1024, and a trainer 1025. The storage 1010 stores training data 1032 and a trained third learning model 1034 in addition to a control program 1031, which is a program for executing various types of controls of the information processing device 1001.

### Acquirer 1021

The acquirer 1021 acquires input information including a first image of the subject. The input information is data input to the estimator 1023. The input information includes a first image showing the trabecular bone of the subject and/or a virtual image that represents a color shading in the first image for each predetermined area. In the following description, the first image showing the trabecular bone of the subject may be referred to as a first medical image, and the virtual image that represents the color shading in the medical image for each predetermined area may be referred to as a first virtual image. The acquirer 1021 may acquire the first medical image and/or the first virtual image from the image management device 5A. The acquirer 1021 may acquire a first virtual image generated by a virtual image generation device other than the image management device 5A using the first medical image from the virtual image generation device. In addition to the first medical image and the first virtual image, the acquirer 1021 may acquire, as input information, attribute information of the subject from the electronic medical record management device 6, may acquire a test numerical value of the subject from the test numerical value management device 1005, or may acquire a diagnostic result of the subject from the diagnostic result management device 1006.

### Estimator 1023

The estimator 1023 inputs the input information acquired by the acquirer 1021 into the third learning model 1034, thereby estimating third estimation information, which is bone information regarding the subject's bone state. In the present embodiment, an example will be described in which the estimator 1023 estimates future bone information of a subject. However, past bone information of a subject may also be estimated. Here, the third learning model 1034 is trained in advance using the training data 1032. The training data 1032 is data including medical information of a predetermined person. The predetermined person may be a patient suffering from a bone-related disease or a person not suffering from a disease. The medical information included in the training data 1032 includes information including a second medical image showing the trabecular bone of a predetermined person and trabecular bone information regarding the state of the trabecular bone of the predetermined person and/or a second virtual image that represents a color shading in the second medical image for each predetermined area. In the following description, the medical image showing the trabecular bone of the predetermined person may be referred to as a second medical image, and the virtual image that represents the color shading in the second medical image for the predetermined area may be referred to as a second virtual image.

The estimator 1023 may estimate future bone information of the subject by inputting input information including the first medical image and/or the first virtual image into the third learning model 1034. The estimator 1023 may estimate future bone information of the subject by (1) inputting the medical information including the first medical image into the third learning model 1034 trained using training data including at least information including a second medical image showing the trabecular bone of a predetermined person and trabecular bone information regarding the state of the trabecular bone of the predetermined person, or (2) inputting medical information including the first virtual image into the third learning model 1034 trained using training data including at least a second virtual image that represents a color shading in the second medical image for each predetermined area. The estimator 1023 may input the above input information into the third learning model 1034, and thereby estimate, as the bone information, at least one selected from the group consisting of a trabecular bone score, a probability of a fracture occurrence, a likelihood of osteoporosis, a drug efficacy, an incident occurrence, a bone density, a trabecular number, a trabecular spacing, and a bone connectivity density. Alternatively, bone strength may be analyzed based on the state of cortical bone and/or cancellous bone and the resulting data may be used as bone information.

The third learning model 1034 is an operation model used by the estimator 1023 in performing an operation, based on the input data. The third learning model 1034 is generated by the trainer 1025 executing machine learning using the training data 1032, which will be described below, on an untrained neural network. Here, the third learning model 1034 may also be applied to an animal other than a human. In such a case, the "predetermined person" in the training data 1032 may be a biological species of the same type as the "subject." That is, the information processing device 1001 according to the present disclosure can also estimate bone information regarding the state of the bones of an animal other than a human. A specific example of the training data 1032, a configuration of the neural network, and the training processing will be described below.

### Outputter 1024

The outputter 1024 transmits information estimated by the estimator 1023 to a terminal device 1030. The information processing device 1001 may include a display (not illustrated). In such a case, the outputter 1024 causes the display to display the information estimated by the estimator 1023.

### Trainer 1025

The trainer 1025 controls training processing on the untrained neural network. The trainer 1025 creates a trained neural network functioning as the estimator 1023 by executing training processing on the untrained neural network. Training data 1032 (described below) is used for such training. A specific example of training performed by the trainer 1025 will be described below.

### Configuration of Estimator 1023

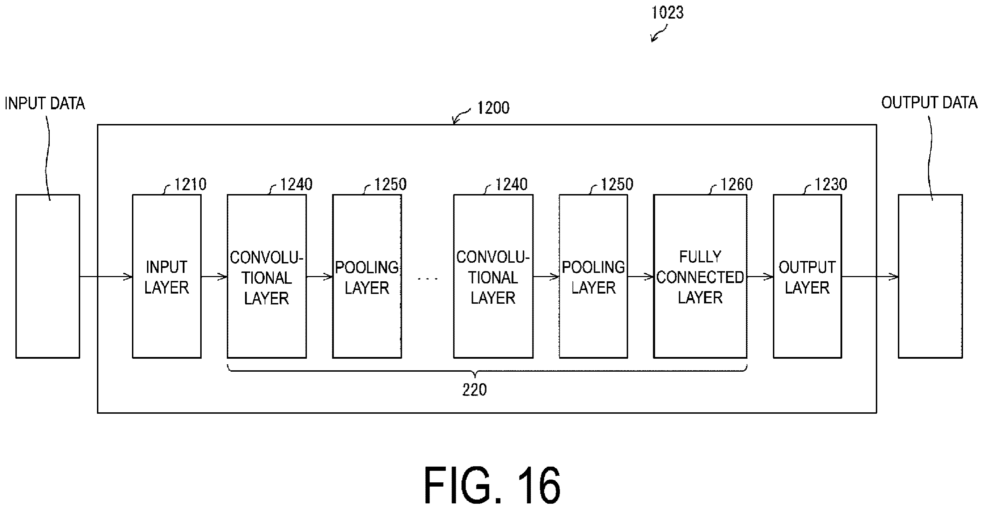

A configuration of the estimator 1023 will be described below with reference to FIG. 16. The configuration illustrated in FIG. 16 is merely an example and the configuration of the estimator 1023 is not limited thereto.

As illustrated in FIG. 16, the estimator 1023 performs operations based on the third learning model 1034 on input data input to an input layer 1210 to output output data from an output layer 1230. The output data is future bone information of the subject. The future bone information may be, for example, at least one selected from the group consisting of a trabecular bone score, a probability of a fracture occurrence, a likelihood of osteoporosis, a drug efficacy, an incident occurrence, a bone density, a trabecular number, a trabecular spacing, a trabecular width, a trabecular orientation, and a bone connectivity density.

The estimator 1023 in FIG. 16 includes a neural network 200 having the input layer 1210 and the output layer 1230. FIG. 16 illustrates a case where the neural network 1200 is a CNN. As illustrated in FIG. 16, the neural network 1200 includes, for example, the input layer 1210, a hidden layer 1220, and the output layer 1230. The hidden layer 1220 is also called an intermediate layer. The hidden layer 1220 includes, for example, a plurality of convolutional layers 1240, a plurality of pooling layers 1250, and a fully connected layer 1260. In the neural network 1200, the fully connected layer 1260 exists before the output layer 1230. In the neural network 1200, the convolutional layers 1240 and the pooling layers 1250 are alternately arranged between the input layer 1210 and the fully connected layer 1260. Note that the configuration of the neural network 1200 is not limited to the example illustrated in FIG. 16. For example, the neural network 1200 may include one convolutional layer 1240 and one pooling layer 1250 between the input layer 1210 and the fully connected layer 1260. The neural network 1200 may be a neural network other than a convolutional neural network.

The neural network 1200 is not limited to the CNN, and may be an LSTM. For example, the neural network 1200, which is a neural network, may be a ConvLSTM network in which a CNN and an LSTM are combined. In this case, the input layer can extract a feature for a change in the input data. The output layer can calculate a new feature based on the feature extracted in the input layer, the temporal change in the input data, and the initial value of the input data. Each of the input layer and the output layer includes a plurality of LSTM layers. Each of the input layer and the output layer may include three or more LSTM layers.

### Training Processing by Trainer 1025

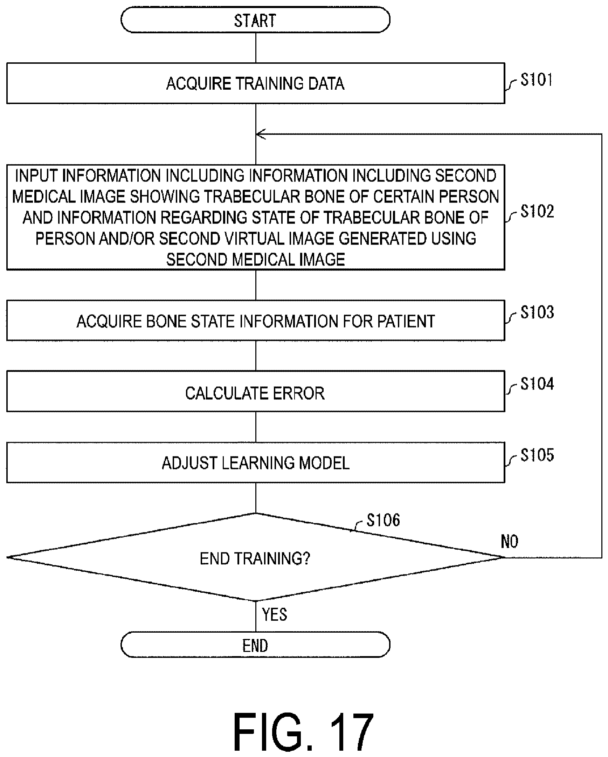

The training processing for generating the third learning model 1034 will be described below with reference to FIG. 17. FIG. 17 is a flowchart illustrating an example of a flow of training processing by the trainer 1025.

The trainer 1025 acquires the training data 1032 from the storage 1010 (step S101). The training data 1032 includes information including a second medical image showing the trabecular bone of a predetermined person and trabecular bone information regarding the state of the trabecular bone of the predetermined person and/or a second virtual image, and data regarding the state of the trabecular bone of the predetermined person a predetermined time after the second medical image is captured, and includes an explanatory variable and a response variable to be input into the input layer 1210. Here, the explanatory variable is information including a second medical image showing the trabecular bone of a predetermined person and trabecular bone information regarding the state of the trabecular bone of the predetermined person and/or a second virtual image, and the response variable is data regarding the state of the trabecular bone of the predetermined person a predetermined time after the second medical image is captured.

Next, the trainer 1025 inputs information including information including a second medical image showing the trabecular bone of a certain person (referred to as a person A) and trabecular bone information regarding the state of the trabecular bone of the predetermined person and/or a second virtual image into the input layer 210 (step S102).

The trainer 1025 acquires output data regarding the bone information of the person A from the output layer 1230 (step S103). The output data contains the same contents as the response variable of the training data 1032. In FIG. 17, the order of step S102 and step S103 may be reversed. Alternatively, in FIG. 17, step S102 and step S103 may be executed simultaneously.

The trainer 1025 acquires a response variable for the person A included in the training data 1032. The trainer 1025 compares the output data acquired in step S103 with the response variable for the person A, calculates an error (step S104), and adjusts the third learning model 1034 being trained so as to reduce the error (step S105).

Any known method is applicable to the adjustment of the learning model being trained. For example, backpropagation may be employed as a method for adjusting the learning model. The adjusted learning model becomes a new learning model, and in subsequent operations, the estimator 1023 uses the new learning model. In an adjustment stage of the learning model, the parameters (for example, a filter coefficient and a weighting factor) used in the estimator 1023 may be adjusted.

If the error is not within a predetermined range, and if explanatory variables for all the persons contained in the training data 1032 are not input (NO in step S106), the trainer 1025 returns to step S102 and repeats the training processing. If the error is within the predetermined range, and if the explanatory variables for all the persons contained in the training data 1032 are input (YES in step S106), the trainer 1025 ends the training processing.

When the above-described training processing is employed, the estimator 1023 can estimate future bone information of the subject from medical information including a first medical image showing the trabecular bone of the subject and/or a first virtual image that represents a color shading in the first medical image for each predetermined area. When the training data 1032 includes a plurality of first medical images and/or first virtual images of the same person, a third learning model 1034 may be constructed based on the temporal change in the features of characteristic regions. Thus, future bone information of the subject can be estimated in consideration of the temporal change in the state of the trabecular bone, thereby improving the accuracy of the estimation.

### Processing Performed by Information Processing Device 1001

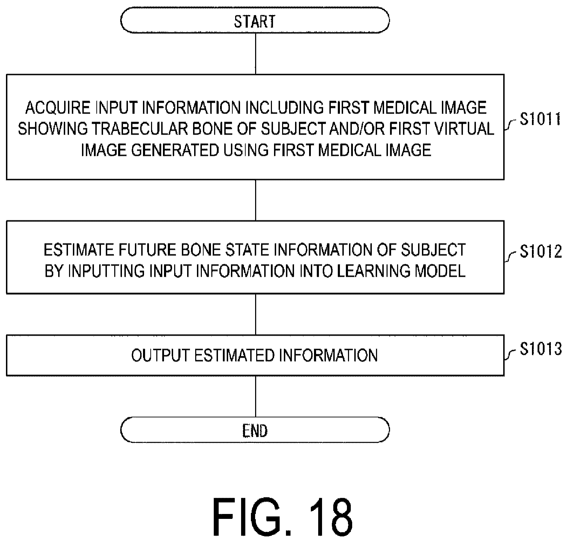

A flow of processing performed by the information processing device 1001 will be described below with reference to FIG. 18. FIG. 18 is a flowchart illustrating an example of a flow of processing performed by the information processing device 1001.

First, the acquirer 1021 acquires medical information including a first medical image showing the trabecular bone of the subject and/or a first virtual image that represents a color shading in the first medical image for each predetermined area (step S1011: acquiring step).

Next, the estimator 1023 estimates future bone information of the subject by inputting the acquired medical information into the third learning model 1034 (step S1012: estimating step).

The outputter 1024 outputs each piece of information including the bone information estimated in step S1012 (step S1013: outputting step).

According to this configuration, the information processing device 1001 and the information processing system 1100a estimate future bone information of the subject from medical information including a first medical image showing the trabecular bone of the subject and/or a first virtual image that represents a color shading in the first medical image for each predetermined area. Here, the first medical image shows a trabecular bone, which is an internal structure of a bone. Therefore, the estimation result output from the third learning model 1034 when the estimator uses, as input information, the first medical image or the first virtual image generated using the first medical image reflects the internal structure of the bone, and therefore has high estimation accuracy. That is, the information processing device 1001 and the information processing system 1100a can estimate bone information regarding the future bone state of the subject with high accuracy.

In the above description, the information processing device 1001 estimates bone information regarding the future bone state of a subject, but the information processing device 1001 according to the present embodiment is not limited thereto. The information processing device 1001 may estimate bone information regarding the current bone state of the subject. In this case, the trainer 1025 may generate the third learning model 1034 using, as the response variable, data regarding the state of the trabecular bone of the predetermined person at the point in time at which the second medical image is captured. Thus, the estimator 1023 can estimate bone information regarding the current bone state of the subject by inputting medical information including the first medical image and/or the first virtual image into the third learning model 1034.

Here, the bone state changes significantly due to menopause. Therefore, when the estimator 1023 estimates bone information regarding the future bone state of a subject, and the subject is a woman who is not menopausal at the current point in time, the estimator 1023 may output a first estimation result on the assumption that the subject is menopausal and/or a second estimation result on the assumption that the subject is not menopausal. Accordingly, by taking into account both cases before and after menopause, an estimation result with higher accuracy may be output. When the subject is a woman who is not menopausal at the current point in time, the estimator 1023 may simultaneously output the first estimation result on the assumption that the subject is menopausal and the second estimation result on the assumption that the subject is not menopausal.

### Fifth Embodiment

### Configuration of Information Processing System 1100b

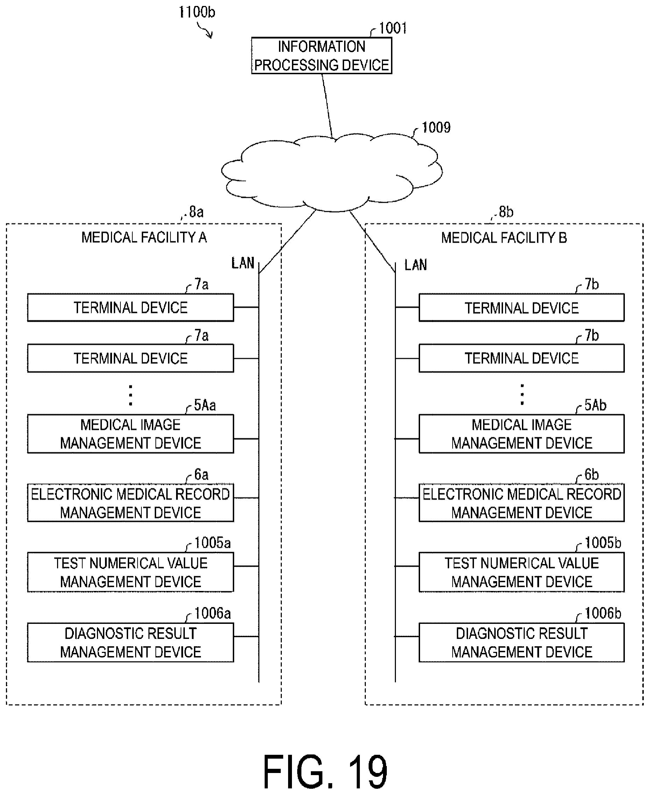

The information processing device 1001 may be communicatively connected to LANs each disposed in the corresponding one of the plurality of medical facilities 8 via a communication network 1009, instead of a computer installed in the predetermined medical facility 8. FIG. 19 is a diagram illustrating a configuration example of an information processing system 1100b according to another aspect of the present disclosure.

In addition to one or more terminal devices 7a, an image management device 5Aa, an electronic medical record management device 6a, a test numerical value management device 1005a, and a diagnostic result management device 1006a may be communicatively connected to the LAN in the medical facility 8a. In addition to a terminal device 7b, an image management device 5Ab, an electronic medical record management device 6b, a test numerical value management device 1005b, and a diagnostic result management device 1006b may be communicatively connected to the LAN in the medical facility 8b. In the present disclosure, when no particular distinction is made between the medical facilities 8a and 8b, each of the medical facilities 8a and 8b is referred to as a "medical facility 8." When no particular distinction is made between the terminal devices 7a and 7b, the image management devices 5Aa and 5Ab, the electronic medical record management devices 6a and 6b, the test numerical value management devices 1005a and 1005b, and the diagnostic result management devices 1006a and 1006b, they will be referred to as the "terminal device 7," the "image management device 5A," the "electronic medical record management device 6," the "test numerical value management device 1005," and the "diagnostic result management device 1006," respectively.

FIG. 19 illustrates an example in which the LANs in the medical facilities 8a and 8b are connected to the communication network 1009. The information processing device 1001 is not limited to the configuration illustrated in FIG. 19 as long as the information processing device 1001 is communicatively connected to the image management device 5A, the electronic medical record management device 6, the test numerical value management device 1005, and the diagnostic result management device 1006 in each medical facility via the communication network 1009. For example, the information processing device 1001 may be installed in the medical facility 8a or in the medical facility 8b.

In the information processing system 1100b employing such a configuration, the information processing device 1001 can acquire a first image of a subject Pa examined in the medical facility 8a, the attribute information, the test numerical value, and the diagnostic result, from the image management device 5Aa, the electronic medical record management device 6a, the test numerical value management device 1005a, and the diagnostic result management device 1006a in the medical facility 8a, respectively. The information processing device 1001 transmits the estimation result of the future bone information of the subject Pa to the terminal device 7a installed in the medical facility 8a. Similarly, the information processing device 1001 transmits the future bone information of a subject Pb to the terminal device 7b.

### Supplementary Note

The information processing device 1001 need not include all the functional blocks included in the controller 1002 of the information processing device 1001. For example, in the information processing system 1100a, the terminal device 7 may have the function of the acquirer 1021, and the information processing device 1001 may receive the input information from the terminal device 7. Similarly, in the information processing system 1100b, the information processing device 1001 may receive input information from the terminal device 7.

For example, in the information processing systems 1100a and 1100b, the function of the trainer 1025 may be configured to install the third learning model 1034, which has been trained by a computer other than the information processing device 1001, into the information processing device 1001. For example, in the information processing systems 1100a and 1100b, the terminal device 1030 or a computer other than the information processing device 1001 may have the function of the trainer 1025. In the information processing systems 1100a and 1100b, the terminal device 1030 or a computer different from the information processing device 1001 may have the function of the estimator 1023.

### Example of Implementation Using Software

The functions of the information processing devices 1, 1a, and 1001 (hereinafter, referred to as the "device") can be implemented by a program for causing a computer to function as the device and for causing the computer to function as individual control blocks (particularly, individual components included in the controllers 2, 2a, and 1002) of the device.

In such a case, the device includes a computer including at least one control device (for example, a processor) and at least one storage device (for example, a memory) as hardware for executing the above program. By executing the program by the control device and the storage device, the functions described in the embodiments are implemented.

The program may be recorded on one or more computer-readable non-transitory recording media. The recording media may be or may not be included in the device. In the latter case, the program may be supplied to the device via any wired or wireless transmission medium.

Some or all of the functions of the control blocks can be implemented by logic circuits. For example, an integrated circuit in which logic circuits functioning as the control blocks are formed is also included in the scope of the present invention. In addition to this, for example, a quantum computer can implement the functions of the control blocks.

The processing described in the embodiments may be executed by artificial intelligence (AI). In such a case, the AI may operate on the control device or may operate on another device (such as an edge computer or a cloud server).

The invention according to the present disclosure has been described above based on various drawings and examples. However, the invention according to the present disclosure is not limited to each embodiment described above. That is, the invention according to the present disclosure can be modified in various ways within the scope described in the present disclosure, and embodiments obtained by combining technical means disclosed in the different embodiments as appropriate are also included in the technical scope of the invention according to the present disclosure. In other words, it should be noted that a person skilled in the art can easily make various variations or modifications based on the present disclosure. It should also be noted that these variations or modifications are included in the scope of the present disclosure.

### Conclusion

An information processing system according to a first aspect of the present disclosure includes an acquirer configured to acquire input information including a first image showing at least a part of a target bone of a subject in which a first implant is placed, a first estimator configured to input the input information into a first learning model trained using first training data including a second image showing at least a part of a bone of an animal including a person, and bone information regarding at least any of a bone density, a bone mass, and a bone quality of the bone, and estimate first estimation information regarding at least any of a bone density, a bone mass, and a bone quality of the target bone, and an outputter configured to output the first estimation information.

In the information processing system according to a second aspect of the present disclosure, in the first aspect, the second image may include an image showing at least a part of a bone in which a second implant is placed.

In the information processing system according to a third aspect of the present disclosure, in the first or second aspect, the first estimation information may include information regarding at least any of a bone density, a bone mass, and a bone quality for a partial region of the target bone in which the first implant is placed.

In the information processing system according to a fourth aspect of the present disclosure, in any one of the first to third aspects, the first estimation information may include information regarding at least any of a bone density, a bone mass, and a bone quality of a plurality of sites including a site proximate to the first implant in the target bone.

In the information processing system according to a fifth aspect of the present disclosure, in any one of the first to fourth aspects, the first implant may be an artificial hip joint implant including a stem, the target bone may be a femur in which the stem is placed, and the first estimator may estimate a plurality of pieces of the first estimation information within a region of the femur classified by Gruen classification.

In the information processing system according to a sixth aspect of the present disclosure, in the fifth aspect, the first estimation information may include the first estimation information within a proximal region of the femur in which the stem is placed.

In the information processing system according to a seventh aspect of the present disclosure, in any one of the first to fourth aspects, the first implant may be an artificial hip joint implant including a cup, the target bone may be an acetabulum in which the cup is placed, and the first estimator may estimate a plurality of pieces of the first estimation information within a region of the acetabulum classified by Charnley classification.

In the information processing system according to an eighth aspect of the present disclosure, in any one of the first to seventh aspects, the information processing system may further include a second estimator configured to input the input information into a second learning model trained using second training data including a third image showing at least a part of a bone of an animal including a person, and progress information regarding an event that has occurred due to placement of a third implant in the bone, and estimate second estimation information regarding an event that is likely to occur in the subject due to the placement of the first implant, in which the outputter may transmit the second estimation information.

In the information processing system according to a ninth aspect of the present disclosure, in the eighth aspect, the event may include at least any of loosening of the placed first implant, a fracture of the bone in which the first implant is placed, dislocation of a joint associated with the bone in which the first implant is placed, infection of a region including the bone in which the first implant is placed, and a fracture of the first implant.

In the information processing system according to a tenth aspect of the present disclosure, in any one of the first to ninth aspects, the input information may include attribute information of the subject and/or surgical information regarding a placement surgery in which the first implant is placed in the target bone.

In the information processing system according to an eleventh aspect of the present disclosure, in the tenth aspect, the attribute information may include at least any of an age, a sex, a height, a weight, a race, a menopause status, a presence or absence of a fracture, the number of fractures, a location of the fracture, a history of fractures, information regarding lifestyle habits, information regarding a medication being taken, and information indicating a result of a blood test of the subject.

In the information processing system according to a twelfth aspect of the present disclosure, in the tenth aspect, the surgical information may include at least any of a model of the first implant placed in the target bone, a size of the first implant, a surgical procedure for the placement surgery, a surgical time for the placement surgery, an amount of bleeding during the placement surgery, information indicating a medical facility in which the placement surgery has been performed, and information indicating a surgeon for the placement surgery.

In the information processing system according to a thirteenth aspect of the present disclosure, in any one of the first to twelfth aspects, the first implant may include at least any of an artificial joint, a spinal implant, a trauma implant, a plastic implant, and a dental implant.

In the information processing system according to a fourteenth aspect of the present disclosure, in the thirteenth aspect, the artificial joint may be at least any of an artificial hip joint, an artificial knee joint, an artificial shoulder joint, an artificial elbow joint, an artificial ankle joint, and an artificial finger joint, the spinal implant may be at least any of an instrumentation, a cage, an artificial intervertebral disc, and an artificial vertebral body, the trauma implant may be at least any of a plate, a screw, and a nail, and the plastic implant may be a skull plate and/or a nasal bone prosthesis.

A method for controlling an information processing system according to a fifteenth aspect of the present disclosure includes acquiring input information including a first image showing at least a part of a target bone of a subject in which a first implant is placed, and inputting the input information into a learning model trained using first training data including a second image showing at least a part of a bone of an animal including a person, and bone information regarding at least any of a bone density, a bone mass, and a bone quality of the bone, estimating first estimation information regarding at least any of a bone density, a bone mass, and a bone quality of the target bone, and outputting the first estimation information.

A control program of an information processing device according to a sixteenth aspect of the present disclosure is a control program for causing a computer to function as the information processing system described in any one of the first to fourteenth aspects, in which the control program causes the computer to function as the acquirer, the first estimator, and the outputter.

A recording medium according to a seventeenth aspect of the present disclosure is a computer-readable recording medium on which the control program described in the sixteenth aspect is recorded.

### REFERENCE SIGNS

1, 1a Information processing device
21 Acquirer
23 First estimator
24 Outputter
26 Second estimator
32 First training data
33 Trained first learning model
34 Trained second learning model
100a, 100b Information processing system
S11 Acquiring step
S12 Estimating step
S13 Outputting step

## Claims

1. An information processing system comprising:
an acquirer configured to acquire input information comprising a first image showing at least a part of a target bone of a subject in which a first implant is placed;
a first estimator configured to input the input information into a first learning model trained using first training data comprising a second image showing at least a part of a bone of an animal comprising a person, and bone information regarding at least any of a bone density, a bone mass, and a bone quality of the bone, and estimate first estimation information regarding at least any of a bone density, a bone mass, and a bone quality of the target bone; and
an outputter configured to output the first estimation information.

2. The information processing system according to claim 1, wherein the second image comprises an image showing at least a part of a bone in which a second implant is placed.

3. The information processing system according to claim 1 or 2, wherein the first estimation information comprises information regarding at least any of a bone density, a bone mass, and a bone quality for a partial region of the target bone in which the first implant is placed.

4. The information processing system according to any one of claims 1 to 3, wherein the first estimation information comprises information regarding at least any of a bone density, a bone mass, and a bone quality of a plurality of sites comprising a site proximate to the first implant in the target bone.

5. The information processing system according to any one of claims 1 to 4, wherein
the first implant is an artificial hip joint implant comprising a stem,
the target bone is a femur in which the stem is placed, and
the first estimator estimates a plurality of pieces of the first estimation information within a region of the femur classified by Gruen classification.

6. The information processing system according to claim 5, wherein the first estimation information comprises the first estimation information within a proximal region of the femur in which the stem is placed.

7. The information processing system according to any one of claims 1 to 4, wherein
the first implant is an artificial hip joint implant comprising a cup,
the target bone is an acetabulum in which the cup is placed, and
the first estimator estimates a plurality of pieces of the first estimation information within a region of the acetabulum classified by Charnley classification.

8. The information processing system according to any one of claims 1 to 7, further comprising a second estimator configured to input the input information into a second learning model trained using second training data comprising a third image showing at least a part of a bone of an animal comprising a person, and progress information regarding an event that has occurred due to placement of a third implant in the bone, and estimate second estimation information regarding an event that is likely to occur in the subject due to the placement of the first implant,
wherein
the outputter transmits the second estimation information.

9. The information processing system according to claim 8, wherein the event comprises at least any of loosening of the placed first implant, a fracture of the bone in which the first implant is placed, dislocation of a joint associated with the bone in which the first implant is placed, infection of a region comprising the bone in which the first implant is placed, and a fracture of the first implant.

10. The information processing system according to any one of claims 1 to 9, wherein the input information comprises attribute information of the subject and/or surgical information regarding a placement surgery in which the first implant is placed in the target bone.

11. The information processing system according to claim 10, wherein the attribute information comprises at least any of an age, a sex, a height, a weight, a race, a menopause status, a presence or absence of a fracture, the number of fractures, a location of the fracture, a history of fractures, information regarding lifestyle habits, information regarding a medication being taken, and information indicating a result of a blood test of the subject.

12. The information processing system according to claim 10, wherein the surgical information comprises at least any of a model of the first implant placed in the target bone, a size of the first implant, a surgical procedure for the placement surgery, a surgical time for the placement surgery, an amount of bleeding during the placement surgery, information indicating a medical facility in which the placement surgery has been performed, and information indicating a surgeon for the placement surgery.

13. The information processing system according to any one of claims 1 to 12, wherein the first implant comprises at least any of an artificial joint, a spinal implant, a trauma implant, a plastic implant, and a dental implant.

14. The information processing system according to claim 13, wherein
the artificial joint is at least any of an artificial hip joint, an artificial knee joint, an artificial shoulder joint, an artificial elbow joint, an artificial ankle joint, and an artificial finger joint,
the spinal implant is at least any of an instrumentation, a cage, an artificial intervertebral disc, and an artificial vertebral body,
the trauma implant is at least any of a plate, a screw, and a nail, and
the plastic implant is a skull plate and/or a nasal bone prosthesis.

15. A method for controlling an information processing system, the method comprising:
acquiring input information comprising a first image showing at least a part of a target bone of a subject in which a first implant is placed; and
inputting the input information into a learning model trained using first training data comprising a second image showing at least a part of a bone of an animal comprising a person, and bone information regarding at least any of a bone density, a bone mass, and a bone quality of the bone, estimating first estimation information regarding at least any of a bone density, a bone mass, and a bone quality of the target bone, and outputting the first estimation information.

16. A control program for causing a computer to function as the information processing system described in any one of claims 1 to 14, wherein the control program causes the computer to function as the acquirer, the first estimator, and the outputter.

17. A computer-readable recording medium on which the control program described in claim 16 is recorded.
